# EUROPÄISCHE PATENTSCHRIFT

(11) **EP 3 129 351 B1**
(45) Veröffentlichungstag und Bekanntmachung des Hinweises auf die Patenterteilung: **10.04.2019**
(21) Anmeldenummer: 15715731.4
(22) Anmeldetag: 13.04.2015
(51) Int. Cl.: C07C 263/10, C07C 265/14, C08G 18/76, C08G 18/38, G02B 1/04, C08G 18/75, C08G 18/24, C08G 18/71, C08L 75/04

(54) **VERFAHREN ZUR HERSTELLUNG VON XYLYLENDIISOCYANATEN IN DER GASPHASE**
METHOD FOR MANUFACTURING XYLYLENE DIISOCYANATES IN THE GASEOUS PHASE
PROCÉDÉ DE FABRICATION DE DIISOCYANATES DE XYLYLÈNE EN PHASE GAZEUSE

(30) Priorität: 11.04.2014 EP 14164345
(43) Veröffentlichungstag der Anmeldung: 15.02.2017
(73) Patentinhaber: Covestro Deutschland AG, 51373 Leverkusen (DE)
(72) Erfinder: HALPAAP, Reinhard, 51519 Odenthal (DE); WEBER, Ralph, 42799 Leichlingen (DE); SANDERS, Josef, 51375 Leverkusen (DE); RICHTER, Frank, 51373 Leverkusen (DE); SCHÄDLICH, Elsa, Karoline, 53117 Bonn (DE); SCHYMURA, Armin, 41469 Neuss (DE); EHRIG, Martin, 51373 Leverkusen (DE); HOCH, Sebastian, 79104 Freiburg im Breisgau (DE); MALEIKA, Robert, 40589 Düsseldorf (DE)
(74) Vertreter: Levpat
(86) Internationale Anmeldenummer: PCT/EP2015/057956
(87) Internationale Veröffentlichungsnummer: WO 2015/155365

(56) Entgegenhaltungen:
- US-A- 3 424 780
- US-A- 5 306 799
- US-A- 5 391 683
- US-A- 5 679 839

## Beschreibung

Die Erfindung betrifft ein Verfahren zur Herstellung der isomeren 1,3- und/oder 1,4-Xylylendiisocyanate (1,3-XDI, bzw. 1,4-XDI, einzeln oder in Mischung auch als XDI bezeichnet) durch Umsetzung der entsprechenden 1,3- und/oder 1,4-Xylylendiamine (1,3-XDA, bzw. 1,4-XDA, einzeln oder in Mischung auch als XDA bezeichnet) mit Phosgen in der Gasphase.

| | |
|---|---|
| 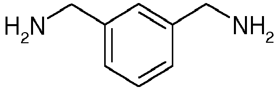 | 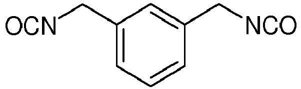 |
| 1,3-Xylylendiamin (1,3-XDA) | 1,3-Xylylendiisocyanat (1,3-XDI) |
| 1,3-Bis(Aminomethyl)benzol | 1,3-Bis(Isocyanatomethyl)benzol |

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen. Man unterscheidet dabei zwischen aromatischen Isocyanaten, bei denen die NCO-Gruppen unmittelbar an einen aromatischen Ring gebunden sind, und aliphatischen oder cycloaliphatischen Isocyanaten, bei denen die NCO-Gruppen an ein aliphatisches sp3-hybridisiertes C-Atom gebunden sind. Bei XDI handelt es sich um ein aliphatisches Diisocyanat mit benzylisch gebundenen NCO-Gruppen sehr spezieller Reaktivität.

Aufgrund ihrer hohen Reaktivität im Vergleich zu aliphatischen Polyisocyanaten und der im Vergleich zu Polyisocyanaten mit aromatisch gebundenen NCO-Gruppen günstigeren toxikologischen Eigenschaften wird XDI bzw. werden XDI-Polyisocyanate vorteilhaft zur Verklebung von Lebensmittelverpackungen eingesetzt. Wegen seines hohen Brechungsindex bei gleichzeitig guter Lichtstabilität wird insbesondere das 1,3-Isomer zur Herstellung von optischen Linsen und Brillengläsern verwendet. Die US 5,306,799 offenbart eine auf XDI basierende Zusammensetzung für eine Kunststofflinse.

Die Herstellung von XDI in der Flüssigphase ist, beispielsweise aus der US 3,424,780, bekannt. In EP 0 384 463 B1 wird ein Verfahren zur Herstellung von XDI beansprucht dadurch gekennzeichnet, dass XDA-Hydrochlorid in einer ersten Reaktionsstufe bei oder unterhalb 30 °C in einem Ester als Lösungsmittel he rgestellt wird und die Phosgenierung des Amin-Hydrochlorids in einem zweiten Reaktionsschritt bei 120-170 °C erfolgt. Ziel dieser speziellen Reaktionsführung ist es, den Gehalt des als Nebenprodukt entstehenden Chlormethyl-benzylisocyanats niedrig zu halten.

US 5,523,467 beansprucht ein Verfahren zur Herstellung von aliphatischen Polyisocyanaten, dadurch gekennzeichnet, dass aliphatische Polyamine, ihre Hydrochloride oder Carbonate in einem inerten Lösungsmittel zunächst mit Phosgen umgesetzt werden und die Reaktion dann unter zusätzlichem Einleiten eines Inertgases fortgesetzt wird. Unter den Bedingungen der Inertgaseinleitung kann der Phosgenüberschuss bei der Herstellung des XDI niedriger gehalten werden und die Bildung von unerwünschtem Teer während der Umsetzung vermindert werden. Nachteilig bei diesem Verfahren ist, dass es sich um einen mehrstufigen Batchprozess handelt, der in hoher Verdünnung in einem relativ hoch siedenden Lösungsmittel durchgeführt wird und der lange Reaktionszeiten erfordert. Das aufwendige Herstellverfahren des XDI ist komplex, energieintensiv und daher teuer. EP 1 908 749 A1 beschreibt die Phosgenierung von Aminhydrochloriden, besonders des 1,3-XDA Hydrochlorids, wobei die Aminhydrochloride unter Überdruck hergestellt werden, um die Teilchengröße in der Suspension zu kontrollieren, um die Viskosität des Slurries zu senken und um während der Phosgenierung wenig Nebenkomponenten, insbesondere wenig 3-Chlormethyl-benzylisocyanat zu erzeugen. Die heterogene Phosgenierreaktion der Salze des XDA in hoher Verdünnung in speziellen Lösungsmitteln und unter Einhaltung einer definierten Reaktionsführung, um die Bildung der unerwünschten Nebenkomponente 3-, bzw. 4-Chlormethyl-benzylisocyanat (3-Chlor-xylylenisocyanat oder 3-Cl-XI; 4-Chlor-xylylenisocyanat oder 4-Cl-XI, einzeln oder in Mischung auch als Cl-XI bezeichnet) oder Teerbildung zu vermeiden, ist schwierig zu realisieren und ist von hohen Ausbeuteverlusten begleitet.

Alle diese Schwierigkeiten stehen bislang einer breiteren Verwendung des XDI entgegen.

Eine besonders wirtschaftliche Möglichkeit zur Herstellung von Isocyanaten ist die Umsetzung der entsprechenden Amine mit Phosgen in der Gasphase.

In der EP 0 289 840 B1 wird ein Verfahren zur Herstellung von (cyclo)aliphatischen Diisocyanaten durch Phosgenierung der entsprechenden, dampfförmigen (cyclo)aliphatischen Diamine bei 200°C bis 600°C beschrieben. Phosgen wird in stöchiometrischem Überschuss zugeführt. Die überhitzten Ströme von dampfförmigem (cyclo)aliphatischem Diamin bzw. (cyclo)aliphatischem Diamin-Inertgas-Gemisch einerseits und von Phosgen andererseits werden kontinuierlich in einen zylindrischen Reaktionsraum geleitet, dort miteinander vermischt und zur Reaktion gebracht. Die exotherme Phosgenierreaktion wird unter Aufrechthaltung einer turbulenten Strömung durchgeführt.

In EP-A 0 928 785, EP-A 1 319 655, EP-A 1 555 258, EP-A 1 275 639, EP-A 1 275 640, EP-A 1 403 248 und EP-A 1 526 129 werden zu dieser Technologie spezielle Ausführungsformen beschrieben, welche sich allerdings auf den Reaktor an sich und die Reaktionsführung beziehen, ohne näher auf die Verdampfertechnologie, die zur Vorbehandlung der Edukte eingesetzt wird, einzugehen.

In EP 0 593 334 B1 (US 5,391,683) und EP 0 699 657 B1 (US 5,679,839) werden Verfahren zur Herstellung von aromatischen Polyisocyanaten beansprucht. Die Umsetzung von Xylylendiamin neben Toluylendiamin und Phenylendiamin mit Phosgen wird genannt, gemeint ist jedoch in beiden Fällen Diamino-xylol. US 2012/0095255 A beansprucht ein Verfahren zur Herstellung von Isocyanaten, bei dem ein Amin und Phosgen miteinander vermischt und in der Gasphase umgesetzt werden können. In einem Unteranspruch (claim 26) wird auch die Umsetzung von p-Xylylendiamin in einer langen Reihe von Mono-, Di- und Triaminen beansprucht. In keiner dieser Anmeldungen finden sich Hinweise auf die besonderen Schwierigkeiten, die bei der Verdampfung von XDA auftreten bzw. zur Überwindung derselben.

EP 1 754 698 B1 beschreibt ein Verfahren zur Herstellung von Isocyanaten durch Phosgenierung von Aminen in der Gasphase, bei dem zur Flüssigerhitzung, Verdampfung und/oder Gasüberhitzung der Amine ein oder mehrere Wärmetauscher mit einer volumenspezifischen Wärmetauscherfläche für die Aminseite von mindestens 1.000 m²/m³ eingesetzt werden, die zur Strömungsführung der Amine Kanäle aufweisen, die einen hydraulischen Durchmesser von 1.000 bis 10.000 µm besitzen. Auch dieses Verfahren ist nicht zur Phosgenierung von XDA geeignet, da die Kanäle dieser Wärmetauscher aufgrund ihrer kleinen Dimensionen durch die sich bildenden hochviskosen bzw. bei weiterer Temperaturbelastung fest werdenden Xylylen-Polyamine sehr schnell verstopfen.

WO 2013 079517 A1 beschreibt ein spezielles Verfahren zur Herstellung von Isocyanaten durch Gasphasenphosgenierung. In dieser besonderen Ausführungsform wird ein Verfahren zur Herstellung von Isocyanaten durch Phosgenierung der entsprechenden Amine in einem Wirbelschichtreaktor beansprucht, das dadurch gekennzeichnet ist, dass ein Gasstrom, enthaltend das Phosgen, als Wirbelgas genutzt wird und einen inerten Feststoff in der Schwebe hält, und dass ein flüssiger Strom, enthaltend das Amin, in die Wirbelschicht dosiert wird, wobei das Amin teilweise oder vollständig verdampft und mit dem Phosgen unter Erhalt eines Reaktionsgasgemisches, enthaltend das entsprechende Isocyanat, reagiert, das aus dem Wirbelschichtreaktor abgezogen wird. Als Vorteil wird gesehen, dass die Reaktionswärme zur Verdampfung und Überhitzung der Amine in der Wirbelschicht genutzt wird. Dieser Anmeldung ist zu entnehmen, dass zur Gasphasenphosgenierung nur thermisch stabile Amine geeignet sind, die sich nur zu einem relativ geringen Grad bei den notwendigen hohen Verdampfungstemperaturen zersetzen, zu höchstens 2 Mol-%, besonders bevorzugt zu höchstens 1 Mol-% oder ganz besonders bevorzugt zu höchstens 0,5 Mol-%. Zu diesen thermisch stabilen Aminen gehören aliphatische Diamine und aromatische Diamine, die in der Anmeldung beispielhaft aufgeführt sind. Benzylische Amine gehören nicht zu dieser Gruppe, wie nachfolgend anhand eigener Ergebnisse beschrieben.

Wie eigene Versuche aber zeigten, können die Gasphasen-Verfahren nach dem beschriebenen Stand der Technik nicht ohne weiteres zur Phosgenierung von XDA verwendet werden, da diese bereits bei Temperaturen deutlich unterhalb ihrer Siedetemperatur unter Abspaltung von Ammoniak nichtflüchtige Xylylen-Polyamine bilden. Hierdurch kommt es einmal bereits nach kurzer Zeit zu Viskositätsanstieg, Fouling und Verstopfungen im Aminverdampfer sowie zu Fouling durch Ammoniumchlorid-Ablagerung im Reaktor und im nachfolgenden Abgassystem.

Zusammenfassend kann festgestellt werden, dass bisher kein Verfahren aus dem Stand der Technik zur Herstellung von XDI in der Gasphase geeignet ist. Es bestand daher weiterhin ein großer Bedarf an einem einfachen und kostengünstigen Verfahren zur Herstellung von XDI in der Gasphase, das die beschriebenen Nachteile der Verfahren des Standes der Technik vermeidet.

Überraschend wurde nun gefunden, dass XDI trotz der beschriebenen Schwierigkeiten in guten Ausbeuten und ausreichend langen Anlagenlaufzeiten hergestellt werden kann, indem man das entsprechende XDA bei einem Druck von < 1.000 mbar abs. verdampft, wobei die mittlere Verweilzeit im Verdampferkreislauf 5 bis 90 Minuten beträgt, die Temperatur im Umpumpkreislauf der Verdampfung bei 40 bis 190°C liegt und anschließend die um weitere 10-100°K über die Verdampfungstemperatur des XDA unter dem gegebenen Druck erhitzten XDA-Brüden nach bekannten Verfahren der Gasphasenphosgenierung mit Phosgen umsetzt.

Besonders überraschend ist, dass unter diesen drastischen Reaktionsbedingungen das hochempfindliche XDA nicht - wie gemäß der durchgeführten Temperversuche eigentlich zu erwarten wäre - zur Bildung von Nebenprodukten wie z.B. Ammoniak und nichtflüchtigen Xylylenpolyaminen neigt und dass der Gehalt an Cl-XI bereits in der Rohware typischerweise im Bereich zwischen 0,1 bis 0,5 Gew.-% bezogen auf die Summe aus XDI und Cl-XI liegt.

Gegenstand der vorliegenden Erfindung ist ein Verfahren zur Herstellung von 1,3- und/oder 1,4- Xylylendiisocyanat (XDI) durch Phosgenierung von 1,3- und/oder 1,4-Xylylendiamin (XDA) mit Phosgen in der Gasphase dadurch gekennzeichnet, dass das Xylylendiamin/die Xylylendiamine, ggf. unter Zusatz von Stabilisatoren und/oder eines Inertgases,
a) bei < 1.000 mbar abs., vorzugsweise bei 350 bis 900 mbar abs., besonders bevorzugt bei 500 bis 800 mbar abs. verdampft wird/werden, wobei
b) die mittlere Verweilzeit im Verdampferkreislauf 5 bis 90 Minuten, bevorzugt 10 bis 60 Minuten, besonders bevorzugt 10 bis 45 Minuten beträgt,
c) die Temperatur im Umpumpkreislauf der Verdampfung 40 bis 190°C, bevorzugt 60 bis 170°C, besonders bevorzugt 60 bis 150°C beträgt,
   und
d) die um weitere 10-100°K, bevorzugt 20-50°K über die Verdampfungstemperatur des XDA unter dem gegebenen Druck erhitzten XDA-Brüden nach bekannten Verfahren der Gasphasenphosgenierung mit Phosgen umgesetzt werden.

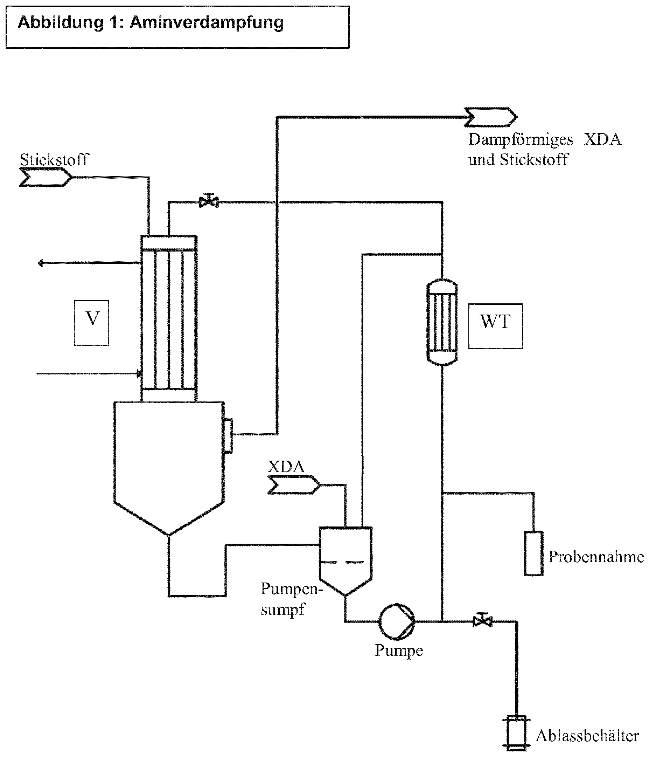

Im erfindungsgemäßen Verfahren wird XDA, ggf. unter Zusatz eines Inertgases wie z.B. Stickstoff bei einem Druck < 1.000 mbar abs. in einem Verdampfer, z.B. in einem Fallrohr-Verdampfer verdampft. Dabei wird der Hold-up im Verdampferkreislauf über die Dimensionierung der Apparate und Rohrleitungen sowie der Verdampferrate so bemessen, dass die mittlere Verweilzeit im Bereich der oben angegebenen Werte liegt. Durch Abkühlung des heißen Rücklaufstroms von nicht verdampftem XDA aus dem Verdampfer (V) mittels eines Wärmetauschers (WT) wird die Temperatur im Umpumpkreislauf der Verdampfung so eingestellt, dass die oben angegebenen Werte nicht unter-, bzw. überschritten werden. Eine mögliche Ausführungsform der erfindungsgemäßen Aminververdampfung wird in Abb. 1 dargestellt.

In einer bevorzugten Ausführungsform wird bzw. werden dem XDA vor der Verdampfung ein oder mehrere Stabilisatoren in Konzentrationen von 50-2.000 ppm (Gewichts-ppm), bevorzugt 100-1.000 ppm, besonders bevorzugt 200-500 ppm, bezogen auf XDA, zugegeben.

Geeignete Stabilisatoren sind z.B. Antioxidantien, z.B. sterisch gehinderte Phenole wie 2,6-Di-tert.butyl-p-kresol (Ionol) und seine Derivate, Säuren z.B. Alkyl-substituierte Benzosulfonsäuren wie Toluolsulfonsäuren, Sulfonamide wie p-Toluolsulfonsäureamid, Phosphite und sek. aromatische Amine. Bevorzugt sind Stabilisatoren, die einen deutlich höheren Siedepunkt aufweisen als XDA und daher im Umpumpkreislauf verbleiben. Als Stabilisatoren bevorzugt werden sterisch gehinderte Phenole. Ein bevorzugter Stabilisator ist z.B. Tinuvin™ 571 der Fa. BASF.

Trotz dieser Maßnahmen kann es im Verdampferkreislauf zu einer Anreicherung von nicht flüchtigen Xylylen-Polyaminen kommen. Es kann daher vorteilhaft sein, durch Ausschleusung einer gewissen XDA-Menge aus dem Verdampferkreislauf ihre Konzentration auf ein solches Maß zu begrenzen, dass die Aminverdampfung, z. B. durch Siedepunktserhöhung, nicht beeinträchtigt wird. Die Ausschleusungsrate wird daher so bemessen, dass die Konzentration an Xylylen-Polyaminen im Verdampferkreislauf < 10%, bevorzugt < 5% liegt.

Selbstverständlich können zur Verdampfung alternativ auch andere Verdampfertypen verwendet werden, die die Ausschleusung gewisser Mengen an nicht flüchtigen Komponenten erlauben, z. B. Dünnschichtverdampfer oder Kurzwegverdampfer.

Das gegebenenfalls vor oder nach der Verdampfung dem XDA zuzugebende Inertgas wird ausgewählt aus der Gruppe umfassend He, Ne, Ar und bevorzugt N₂. Weiterhin können auch die Dämpfe eines inerten Lösungsmittels wie z.B. Xylol, o-Dichlorbenzol (ODB) oder bevorzugt Monochlorbenzol (MCB) zur Verdünnung dem Amin zugegeben werden. Die Menge des gegebenenfalls als Verdünnungsmittel mitverwendeten Inertgases oder Lösungsmitteldampfes ist nicht kritisch, kann jedoch genutzt werden, um die Verdampfungstemperatur des Amins abzusenken.

Das gasförmige, ggf. verdünnte XDA wird vor der Vermischung mit dem Phosgen um weitere 10-100°K, bevorzugt 20-50°K über die Temperatur erhitzt, die im Umpumpkreislauf zur Verdampfung des XDA unter dem dort gegebenen Druck notwendig ist. Dabei werden in einer bevorzugten Ausführungsform des Verfahrens zwischen den Verdampfungs- und Überhitzungssytemen Tröpfchenabscheider eingebaut bzw. besitzen die Verdampfungs- und Überhitzungsapparaturen auch die Funktion eines Tröpfchenabscheiders. Geeignete Tröpfchenabscheider sind z.B. beschrieben in "Droplet Separation", A. Bürkholz, VCH Verlagsgesellschaft Weinheim - New York - Basel - Cambridge 1989. Bevorzugt sind Tröpfchenabscheider, die einen geringen Druckverlust verursachen. Besonders bevorzugt wird das verdampfte XDA über einen Nacherhitzer auf die gewünschte Einsatztemperatur gebracht, der auch als Tröpfchenabscheider fungiert. Ganz besonders bevorzugt hat dieser Nacherhitzer einen Flüssigkeitsablauf, um die stetige Entleerung des Abscheiders zu gewährleisten. Durch die Erzeugung eines im Wesentlichen tröpfchenfreien überhitzten XDA-Stroms vor Eintritt in den Reaktor wird die Reaktorlaufzeit deutlich erhöht.

Das verwendete Phosgen wird vor Einspeisung in den Reaktor auf eine Temperatur von 200-450°C, vorzugsweise 250-350°C erhitzt. Die gegebenenfalls verdünnten, überhitzten Eduktströme werden in einen zylindrischen Reaktionsraum geleitet, dort miteinander vermischt und zur Reaktion gebracht. Die exotherme Phosgenierreaktion wird unter Aufrechthaltung einer turbulenten Strömung durchgeführt.

Die Mengenströme von gasförmigem XDA und Phosgen werden so gewählt, dass der molare Phosgenüberschuss bezogen auf eine Aminogruppe 30 bis 300 %, bevorzugt 60 bis 200 % beträgt.

Geeignete zylindrische Reaktionsräume sind beispielsweise Rohrreaktoren ohne Einbauten und ohne sich bewegende Teile im Innern des Reaktors. Die Rohrreaktoren bestehen im Allgemeinen aus Stahl, Glas, legiertem oder emailliertem Stahl und sind so dimensioniert, dass unter den Verfahrensbedingungen eine vollständige Umsetzung des XDA mit dem Phosgen ermöglicht wird. Die Gasströme werden im Allgemeinen an einem Ende des Rohrreaktors in diesen eingeleitet, wobei diese Einleitung beispielsweise durch an diesem Ende des Rohrreaktors angebrachte Düsen oder durch eine Kombination aus Düse und einem Ringspalt zwischen Düse und Mischrohr erfolgen kann. Das Mischrohr wird ebenfalls bei einer Temperatur innerhalb des Bereichs von 200 bis 450°C, vorzugsweise 250 bis 350°C, gehalten, wobei diese Temperatur gegebenenfalls durch Beheizen des Reaktionsrohrs aufrecht erhalten wird.

Bei der Durchführung des erfindungsgemäßen Verfahrens liegt im allgemeinen der Druck in den Zuleitungen zum Reaktionsraum bei < 1.000 mbar abs., bevorzugt bei 350 bis 900 mbar abs., besonders bevorzugt, bei 500 bis 800 mbar abs. und am Ausgang aus dem Reaktionsraum bei < 950 mbar abs., bevorzugt bei 300 bis 850 mbar abs., besonders bevorzugt 450 bis 750 mbar abs., wobei durch Aufrechterhaltung eines geeigneten Differenzdrucks eine Strömungsgeschwindigkeit innerhalb des Reaktionsraums von 3 bis 100, vorzugsweise 5 bis 75 und besonders bevorzugt 10 bis 60 m/s eingehalten wird. Unter diesen Voraussetzungen herrschen innerhalb des Reaktionsraums im Allgemeinen turbulente Strömungsverhältnisse vor.

Die Verweilzeit des Reaktionsgemischs im Reaktor beträgt 0,1 bis 2 s, vorzugsweise 0,2 bis 1 s und ganz besonders bevorzugt 0,4 bis 0,8 s. Die Verweilzeit berechnet sich aus dem zeitlichen Durchsatz der Eduktströme, der Reaktordimensionierung und den Reaktionsparametern Druck und Temperatur.

Nach der erfolgten Phosgenierungsreaktion im Reaktionsraum wird das den Reaktionsraum kontinuierlich verlassende, gasförmige Gemisch von dem gebildeten Isocyanat befreit. Dies kann beispielsweise mit Hilfe eines inerten Lösungsmittels erfolgen, dessen Temperatur so gewählt wird, dass sie einerseits oberhalb der Zersetzungstemperatur des dem Isocyanat entsprechenden Carbamidsäurechlorids und andererseits unterhalb der Kondensationstemperatur des Isocyanats und vorzugsweise auch des gegebenenfalls in der Dampfform als Verdünnungsmittel mitverwendeten Lösungsmittels liegt, so dass Isocyanat und Hilfslösungsmittel kondensieren bzw. sich in dem Lösungsmittel lösen, während überschüssiges Phosgen, Chlorwasserstoff und gegebenenfalls als Verdünnungsmittel mitverwendetes Inertgas die Kondensationsstufe bzw. das Lösungsmittel gasförmig durchlaufen. Zur selektiven Gewinnung des Isocyanats aus dem den Reaktionsraum gasförmig verlassenden Gemisch besonders gut geeignet sind bei einer Temperatur von 60 bis 200°C, vorzugsweise 90 bis 170°C, gehaltene Lösungsmittel der oben beispielhaft genannten Art, insbesondere technisches Mono-(MCB) und Dichlorbenzol (ODB). Denkbare Methoden der selektiven Kondensation des gebildeten Isocyanats aus dem den Reaktor verlassenden Gasgemisch unter Verwendung derartiger Lösungsmittel sind beispielsweise das Durchleiten des Gasgemisches durch das genannte Lösungsmittel oder das Eindüsen des Lösungsmittels (Lösungsmittelnebel) in den Gasstrom, Quenche.

Das die Kondensationsstufe zur Gewinnung des Isocyanats durchlaufende Gasgemisch wird anschließend in an sich bekannter Weise von überschüssigem Phosgen befreit. Dies kann mittels einer Kühlfalle, Absorption in einem bei einer Temperatur von -10°C bis 8°C gehaltenen inerten Lösungsmittel (z.B. Chlorbenzol, MCB, oder Dichlorbenzol, ODB) oder durch Adsorption und Hydrolyse an Aktivkohle erfolgen. Das die Phosgen-Rückgewinnungsstufe durchlaufende Chlorwasserstoffgas kann in an sich bekannter Weise zur Rückgewinnung des zur Phosgensynthese erforderlichen Chlors recykliert werden.

Die Reindarstellung der Isocyanate erfolgt vorzugsweise durch destillative Aufarbeitung der Lösung des Isocyanats in dem zur Isocyanatkondensation eingesetzten Lösungsmittel.

Die Erfindung betrifft weiterhin 1,3- und/oder 1,4- Xylylendiisocyanat, hergestellt oder herstellbar nach dem erfindungsgemäßen Verfahren. Die beiden vorgenannten Verbindungen unterscheiden sich von 1,3- beziehungsweise 1,4- Xylylendiisocyanat, welches über die bislang eingesetzte Flüssigphasenphosgenierung von XDA erzeugt wurden. Dies wird insbesondere daran deutlich, dass bei der Verwendung von erfindungsgemäß erzeugtem 1,3- und/oder 1,4- Xylylendiisocyanat in einer Polythiourethanformulierung nach der Polymerisation mit einer Polythiolkomponente Polythiourethan-Formkörper erhalten werden, die eine deutlich geringere Trübungsneigung gegenüber solchen Formkörpern aufweisen, die aus der gleichen Polythiourethanformulierung, jedoch mit 1,3- und/oder 1,4- Xylylendiisocyanat aus der Flüssigphasenphosgenierung erzeugt wurden.

Zudem bietet das erfindungsgemäße Verfahren auch prozesstechnische Vorteile. Um den hohen Anforderungen bei optischen Anwendungen zu genügen, sind bislang aufwändige Verfahren für die Herstellung der Rohstoffe vonnöten. So beschreibt die EP-A 1 908 749 ein Verfahren zur Herstellung von XDI, bei dem zunächst das Hydrochlorid des XDA hergestellt und dieses dann unter erhöhtem Druck phosgeniert wird, um entsprechend reine Qualitäten zu erhalten. Demgegenüber erfolgt bei dem erfindungsgemäßen Verfahren die Phosgenierung des XDA direkt.

Entsprechend der vorstehenden Ausführung ist ein weiterer Gegenstand der vorliegenden Erfindung die Verwendung von erfindungsgemäß erzeugtem 1,3- und/oder 1,4-Xylylendiisocyanat zur Herstellung transparenter Polyurethane oder Polythiourethane, insbesondere zur Herstellung transparenter Polyurethan- oder Polythiourethan-Formkörper, wobei die Polythiourethan-Formkörper wegen ihrer guten optischen Eigenschaften besonders bevorzugt sind.

Im Rahmen der vorliegenden Erfindung wird unter dem Begriff transparent verstanden, dass der transparente Körper bei einer Dicke von 2 mm und bei der Normlichtart D65 (definiert in DIN 6173) eine Transmission von ≥ 85 % aufweist. Wobei dieser Transmissionswert bei der optionalen Mitverwendung von UV-Stabilisatoren und Farbstoffen von dem vorstehend genannten Wert von ≥ 85 % abweichen kann.

Die Erfindung betrifft weiterhin auch die erfindungsgemäß erzeugten Polyurethan- oder Polythiourethan-Formkörper an sich, wobei die Polythiourethan-Formkörper wegen ihrer guten optischen Eigenschaften besonders bevorzugt sind.

Eine geeignete Zusammensetzung zur Herstellung transparenter Polythiourethane oder Polythiourethan-Körper kann enthalten oder bestehen aus
A) einer Polyisocyanatkomponente enthaltend oder basierend auf über Gasphasenphosgenierung gemäß der vorliegenden Erfindung hergestelltes oder herstellbares 1,3- und/oder 1,4- Xylylendiisocyanat (XDI) sowie gewünschtenfalls wenigstens ein weiteres Polyisocyanat mit einer Funktionalität an Isocyanatgruppen von wenigstens 2 pro Molekül,
B) einer Thiolkomponente enthaltend oder bestehend aus zumindest ein Polythiol mit einer Funktionalität an Thiolgruppen von wenigstens 2 pro Molekül,
   sowie gegebenenfalls
C) Hilfs- und Zusatzmittel,
wobei das Verhältnis von Isocyanatgruppen zu gegenüber Isocyanaten reaktiven Gruppen 0,5 : 1 bis 2,0 : 1 beträgt.

Unter "basierend auf" wird vorliegend verstanden, dass die Polyisocyanatkomponente auch Präpolymere umfassen kann, die aus dem erfindungsgemäß erzeugten 1,3- und/oder 1,4- Xylylendiisocyanat (XDI) hergestellt sind.

Eine geeignete Zusammensetzung zur Herstellung transparenter Polyurethane oder Polyurethan-Körper kann enthalten oder bestehen aus
A) einer Polyisocyanatkomponente enthaltend oder basierend auf über Gasphasenphosgenierung gemäß der vorliegenden Erfindung hergestelltes oder herstellbares 1,3- und/oder 1,4- Xylylendiisocyanat (XDI) sowie gewünschtenfalls wenigstens ein weiteres Polyisocyanat mit einer Funktionalität an Isocyanatgruppen von wenigstens 2 pro Molekül,
B) einer Polyolkomponente enthaltend oder bestehend aus zumindest ein Polyol mit einer Funktionalität an Hydroxylgruppen von wenigstens 2 pro Molekül,
   sowie gegebenenfalls
C) Hilfs- und Zusatzmittel,
wobei das Verhältnis von Isocyanatgruppen zu gegenüber Isocyanaten reaktiven Gruppen 0,5 : 1 bis 15,0 : 1 beträgt, bevorzugt 0,5 : 1 bis 2,0 : 1.

Auch Systeme mit einer Mischung aus Polyolkomponente und Thiolkomponente liegen im Rahmen der vorliegenden Erfindung.

Die Polyisocyanatkomponente A) kann auch weitere Polyisocyanate enthalten. Im Prinzip können als weitere Polyisocyanate im Rahmen der vorliegenden Erfindung sämtliche an sich bekannte Polyisocyanate eingesetzt werden. Dies sind beispielsweise solche des Molekulargewichtsbereiches 140 bis 400 g/mol, wie z. B. 1,4-Diisocyanatobutan, 1,5-Diisocyanatopentan, 1,6-Diisocyanatohexan (HDI), 1,5-Diisocyanato-2,2-dimethylpentan, 2,2,4- bzw. 2,4,4-Trimethyl-1,6-diisocyanatohexan, 1,8-Diisocyanatooctan, 1,9-Diisocyanato-nonan, 1,10-Diisocyanatodecan, 1,3- und 1,4-Diisocyanatocyclohexan, 1,4-Diisocyanato-3,3,5-trimethylcyclohexan, 1,3-Diisocyanato-2-methylcyclohexan, 1,3-Diisocyanato-4-methyl-cyclohexan, 1-Isocyanato-3,3,5-trimethyl-5-isocyanatomethylcyclohexan (Isophorondiisocyanat; IPDI), 1-Isocyanato-1-methyl-4(3)-isocyanatomethylcyclohexan, 2,4'- und 4,4'-Diiso-cyanatodicyclohexylmethan (H12-MDI), 1,3- und 1,4-Bis(isocyanatomethyl)cyclohexan, 4,4'-Diisocyanato-3,3'-dimethyldicyclohexylmethan, 4,4' Diisocyanato-3,3',5,5'-tetramethyldicyclo-hexylmethan, 4,4'-Diisocyanato-1,1'-bi(cyclohexyl), 2,5-Bis(isocyanatomethyl)-bicyclo[2.2.1]heptan, 2,6-Bis(isocyanatomethyl)-bicyclo[2.2.1] heptan, 4,4'-Diisocyanato-3,3'-dimethyl-1,1'-bi(cyclohexyl), 4,4'-Diisocyanato-2,2',5,5'-tetra-methyl-1,1'-bi(cyclohexyl), 1,8-Diisocyanato-p-menthan, 1,3-Diisocyanatoadamantan, 1,3-Dimethyl-5,7-diisocyanatoadamantan, 1,3- und 1,4-Bis(1-isocyanato-1-methylethyl)-benzol (TMXDI), Bis(4-(1-isocyanato-1-methylethyl)phenyl)-carbonat, 1,3- und 1,4-Phenylendiiso-cyanat, 2,4- und 2,6-Toluylendiisocyanat sowie beliebige Gemische dieser Isomeren, Diphenylmethan-2,4'- und/oder -4,4'-diisocyanat und Naphthylen-1,5-diisocyanat, sowie beliebige Gemische solcher Diisocyanate. Diese Isocyanate können über beliebige Prozesse erzeugt worden sein.

Ebenso kann als weiteres Polyisocyanat über Flüssigphasenphosgenierung erzeugtes 1,3-Bis(isocyanatomethyl)benzol (m-Xylylendiisocyanat, m-XDI), 1,4-Bis(isocyanatomethyl)benzol (p-Xylylendiisocyanat, p-XDI) eingesetzt werden.

Sofern die erfindungsgemäße Zusammensetzung neben dem erfindungsgemäß erzeugten 1,3- und/oder 1,4- Xylylendiisocyanat weitere Polyisocyanate enthält, besteht die Polyisocyanatkomponente zu wenigstens 80 Gew.-% bezogen auf die Polyisocyanatkomponente aus durch Gasphasenphosgenierung hergestelltem Polyisocyanat, insbesondere zu wenigstens 85 Gew.-%, weiter bevorzugt zuwenigstens 90 Gew.-%, besonders bevorzugt zu wenigstens 95 Gew.-% oder gar zu wenigstens 96 oder wenigstens 98 Gew.-%.

Die erfindungsgemäße Zusammensetzung zur Erzeugung von Polythiourethan-Formkörpern enthält ferner eine Thiolkomponente B). Diese beinhaltet oder besteht aus zumindest einem Polythiol mit einer Funktionalität an Thiolgruppen von wenigstens zwei pro Molekül, insbesondere von 2 bis 6, vorzugsweise von 2 bis 4, besonders bevorzugt von 3 bis 4. Dabei können auch Mischungen von Polythiolen unterschiedlicher Funktionalität eingesetzt werden, sodass sich ungeradzahlige mittlere Funktionalitäten ergeben. Unter einem Polythiol werden im Rahmen der vorliegenden Erfindung organische Thiole mit zwei oder mehr Thiolgruppen verstanden. Damit bezieht sich die Silbe "poly" im Wesentlichen auf die Zahl der Thiolgruppen und bedeutet nicht zwangsläufig, dass das Polythiol eine oligomere oder gar polymere Struktur aufweisen muss. Obgleich können die erfindungsgemäß eingesetzten Polythiole durchaus auch ein Polyehter-Grundgerüst, ein Polythiolether-Grundgerüst oder ein gemischtes Grundgerüst aus O-Ether und S-Ether-Einheiten besitzen.

Das Polythiol kann ein mittleres Molekulargewicht von 80 (Methandithiol) bis etwa 12000 g/mol aufweisen, vorzugsweise 250 bis 8000 g/mol.

Als Polythiole eignen sich beispielsweise Methandithiol, 1,2-Ethandithiol, 1,1-Propandithiol, 1,2-Propandithiol, 1,3-Propandithiol, 2,2-Propandithiol, 1,4-Butandithiol, 2,3-Butandithiol, 1,5-Pentandithiol, 1,6-Hexandithiol, 1,2,3-Propantrithiol, 1,1-Cyclohexandithiol, 1,2-Cyclohexandithiol, 2,2-Dimethylpropan-1,3-dithiol, 3,4-Dimethoxybutan-1,2-dithiol und 2-Methylcyclohexan-2,3-dithiol, Thioethergruppen enthaltende Polythiole, wie z. B. 2,4-Dimercaptomethyl-1,5-dimercapto-3-thiapentan, 4-Mercaptomethyl-1,8-dimercapto-3,6-dithiaoctan, 4,8-Dimercaptomethyl-1,11-dimercapto-3,6,9-trithiaundecan, 4,7-Dimercaptomethyl-1,11-dimercapto-3,6,9-trithiaundecan, 5,7-Dimercaptomethyl-1,11-dimercapto-3,6,9-trithiaundecan, 4,5-Bis(mercaptoethylthio)-1,10-dimercapto-3,8-dithiadecan, Tetrakis(mercaptomethyl)methan, 1,1,3,3-Tetrakis(mercaptomethylthio)propan, 1,1,5,5-Tetrakis(mercaptomethylthio)-3-thiapentan, 1,1,6,6-Tetrakis(mercaptomethylthio)-3,4-dithiahexan, 2-Mercaptoethylthio-1,3-dimercaptopropan, 2,3-Bis(mercaptoethylthio)-1-mercaptopropan, 2,2-Bis(mercaptomethyl)-1,3-dimercaptopropan, Bis(mercaptomethyl)sulfid, Bis(mercaptomethyl)disulfid, Bis(mercaptoethyl)sulfid, Bis(mercaptoethyl)disulfid, Bis(mercaptopropyl)sulfid, Bis(mercaptopropyl)disulfid, Bis(mercaptomethylthio)methan, Tris(mercaptomethylthio)methan, Bis(mercaptoethylthio)methan, Tris(mercaptoethylthio)methan, Bis(mercaptopropylthio)methan, 1,2-Bis(mercaptomethylthio)ethan, 1,2-Bis(mercaptoethylthio)ethan, 2-Mercaptoethylthio)ethan, 1,3-Bis(mercaptomethylthio)propan, 1,3-Bis(mercaptopropylthio)propan, 1,2,3-Tris(mercaptomethylthio)propan, 1,2,3-Tris(mercaptoethylthio)propan, 1,2,3-Tris(mercaptopropylthio)propan, Tetrakis(mercaptomethylthio)methan, Tetrakis(mercaptoethylthiomethyl)methan, Tetrakis(mercaptopropylthiomethyl)methan, 2,5-Dimercapto-1,4-dithian, 2,5-Bis(mercaptomethyl)-1,4-dithian und dessen gemäß JP-A 07118263 erhältliche Oligomere, 1,5-Bis(mercaptopropyl)-1,4-dithian, 1,5-Bis(2-mercaptoethylthiomethyl)-1,4-dithian, 2-Mercaptomethyl-6-mercapto-1,4-dithiacycloheptan, 2,4,6-Trimercapto-1,3,5-trithian, 2,4,6-Trimercaptomethyl-1,3,5-trithian und 2-(3-Bis(mercaptomethyl)-2-thiapropyl)-1,3-dithiolan, Polyesterthiole, wie z. B. Ethylenglycol-bis(2-mercaptoacetat), Ethylenglycol-bis(3-mercaptopropionat), Diethylenglycol(2-mercaptoacetat), Diethylenglycol(3-mercaptopropionat), 2,3-Dimercapto-1-propanol(3-mercaptopropionat), 3-Mercapto-1,2-propandiol-bis(2-mercaptoacetat), 3-Mercapto-1,2-propandiol-bis(3-mercaptopropionat), Trimethylolpropan-tris(2-mercaptoacetat), Trimethylolpropan-tris(3-mercaptopropionat), Trimethylolethan-tris(2-mercaptoacetat), Trimethylolethan-tris(3-mercaptopropionat), Pentaerythrit-tetrakis(2-mercaptoacetat), Pentaerythrit-tetrakis(3-mercaptopropionat), Glycerin-tris(2-mercaptoacetat), Glycerin-tris(3-mercaptopropionat), 1,4-Cyclohexandiol-bis(2-mercaptoacetat), 1,4-Cyclohexandiol-bis(3-mercaptopropionat), Hydroxymethylsulfid-bis(2-mercaptoacetat), Hydroxymethylsulfid-bis(3-mercaptopropionat), Hydroxyethylsulfid(2-mercaptoacetat), Hydroxyethylsulfid(3-mercaptopropionat), Hydroxymethyldisulfid(2-mercaptoacetat), Hydroxymethyldisulfid(3-mercaptopropionat), (2-Mercaptoethylester)thioglycolat und Bis(2-mercaptoethylester)thiodipropionat sowie aromatische Thioverbindungen, wie z. B. 1,2-Dimercaptobenzol, 1,3-Dimercaptobenzol, 1,4-Dimercaptobenzol, 1,2-Bis(mercaptomethyl)benzol, 1,4-Bis(mercaptomethyl)benzol, 1,2-Bis(mercaptoethyl)benzol, 1,4-Bis(mercaptoethyl)benzol, 1,2,3-Trimercaptobenzol, 1,2,4-Trimercaptobenzol, 1,3,5-Trimercaptobenzol, 1,2,3-Tris(mercaptomethyl)benzol, 1,2,4-Tris(mercaptomethyl)benzol, 1,3,5-Tris(mercaptomethyl)benzol, 1,2,3-Tris(mercaptoethyl)benzol, 1,3,5-Tris(mercaptoethyl)benzol, 1,2,4-Tris(mercaptoethyl)benzol, 2,5-Toluoldithiol, 3,4-Toluoldithiol, 1,4-Naphthalindithiol, 1,5-Naphthalindithiol, 2,6-Naphthalindithiol, 2,7-Naphthalindithiol, 1,2,3,4-Tetramercaptobenzol, 1,2,3,5-Tetramercaptobenzol, 1,2,4,5-Tetramercaptobenzol, 1,2,3,4-Tetrakis(mercaptomethyl)benzol, 1,2,3,5-Tetrakis(mercaptomethyl)benzol, 1,2,4,5-Tetrakis(mercaptomethyl)benzol, 1,2,3,4-Tetrakis(mercaptoethyl)benzol, 1,2,3,5-Tetrakis(mercaptoethyl)benzol, 1,2,4,5-Tetrakis(mercaptoethyl)benzol, 2,2'-Dimercaptobiphenyl und 4,4'-Dimercaptobiphenyl.

Vorzugsweise ist das Polythiol ausgewählt aus 4-Mercaptomethyl-1,8-dimercapto-3,6-dithiaoctan, , 2,5-Bismercaptomethyl-1,4-dithian, 1,1,3,3-Tetrakis(mercaptomethylthio)propan, 5,7-Dimercaptomethyl-1,11-dimercapto-3,6,9-trithiaundecan, 4,7-Dimercaptomethyl-1,11-dimercapto-3,6,9-trithiaundecan, 4,8-Dimercaptomethyl-1,11-dimercapto-3,6,9-trithiaundecan, Trimethylolpropan-tris(3-mercaptopropionat), Trimethylolethan-tris(2-mercaptoacetat), Pentaerythrit-tetrakis(2-mercaptoacetat) und/ oder Pentaerythrit-tetrakis(3-mercaptopropionat).

Abgesehen von der Thiolkomponente B) kann die erfindungsgemäße Zusammensetzung auch andere, üblicherweise mit Polyisocyanaten reagierende Komponenten enthalten. Hierbei handelt es sich insbesondere um die üblichen aus der Polyurethanchemie bekannten Polyetherpolyole, Polyesterpolyole, Polyetherpolyesterpolyole, Polythioetherpolyole, polymermodifizierte Polyetherpolyole, Pfropfpolyetherpolyole, Polyetherpolyamine, hydroxylgruppenhaltigen Polyacetale und/oder hydroxylgruppenhaltigen aliphatischen Polycarbonate, die üblicherweise ein Molekulargewicht von 106 bis 12000 g/mol, vorzugsweise 250 bis 8000 g/mol aufweisen. Ein breiter Überblick über geeignete Reaktionspartner B) findet sich beispielsweise in N. Adam et al.: "Polyurethanes", Ullmann's Encyclopedia of Industrial Chemistry, Electronic Release, 7th ed., chap. 3.2 - 3.4, Wiley-VCH, Weinheim 2005.

Diese Polyole können in Kombination mit, bevorzugt aber nicht zusammen mit einer Thiolkomponente B) als Polyolkomponente B) in den vorgenannten Zusammensetzungen zur Herstellung von Polyurethan-Formkörpern eingesetzt werden.

Unter einem Polythiourethan wird im Sinne der vorliegenden Erfindung ein Polymer verstanden, bei dem mehr als die Hälfte bis sämtliche der Bindungen zwischen dem Polyisocyanat und der oder den Isocyanat-reaktiven Komponente(n) Thiourethangruppen sind. Es können also zu weniger als die Hälfte andere Bindungen vorhanden sein, wie beispielsweise Urethan- oder Harnstoffbrücken. In diesem Falle enthält die erfindungsgemäße Zusammensetzung noch andere Isocyanat-reaktiven Komponente(n). Diese lediglich optionalen Komponenten werden im Folgenden näher beschrieben.

Geeignete Polyetherpolyole, sofern verwendet, sind beispielsweise solche der in der DE-A 2 622 951, Spalte 6, Zeile 65 - Spalte 7, Zeile 47, oder der EP-A 0 978 523 Seite 4, Zeile 45 bis Seite 5, Zeile 14 genannten Art, sofern sie den oben gemachten Angaben hinsichtlich Funktionalität und Molekulargewicht entsprechen. Besonders bevorzugte Polyetherpolyole B) sind Anlagerungsprodukte von Ethylenoxid und/oder Propylenoxid an Glycerin, Trimethylolpropan, Ethylendiamin und/oder Pentaerythrit.

Geeignete Polyesterpolyole, sofern verwendet, sind beispielsweise solche der in der EP-A 0 978 523 Seite 5, Zeilen 17 bis 47 oder der EP-A 0 659 792 Seite 6, Zeilen 8 bis 19 genannten Art, sofern sie den oben gemachten Angaben entsprechen, bevorzugt solche, deren Hydroxylzahl von 20 bis 650 mg KOH/g beträgt.

Geeignete Polyacetalpolyole, sofern verwendet, sind beispielsweise die bekannten Umsetzungsprodukte einfacher Glykole, wie z. B. Diethylenglykol, Triethylenglykol, 4,4'-Dioxethoxy-diphenyl-dimethylmethan (Addukt von 2 mol Ethylenoxid an Bisphenol A) oder Hexandiol, mit Formaldehyd oder auch durch Polykondensation cyclischer Acetale, wie z. B. Trioxan, hergestellte Polyacetale.

Ebenfalls können Aminopolyether oder Gemische von Aminopolyethern geeignet sein, d. h. Polyether mit gegenüber Isocyanatgruppen reaktionsfähigen Gruppen, die sich zumindest zu 50 Äquivalent-%, vorzugsweise zumindest zu 80 Äquivalent-%, aus primären und/oder sekundären, aromatisch oder aliphatisch gebundenen Aminogruppen und zum Rest aus primären und/oder sekundären, aliphatisch gebundenen Hydroxylgruppen zusammensetzen. Geeignete derartige Aminopolyether sind beispielsweise die in EP-A 0 081 701, Spalte 4, Zeile 26 bis Spalte 5, Zeile 40, genannten Verbindungen. Ebenfalls als Ausgangskomponente E) geeignet sind aminofunktionelle Polyetherurethane oder -harnstoffe, wie sie sich beispielsweise nach dem Verfahren der DE-A 2 948 419 durch Hydrolyse von isocyanatfunktionellen Polyetherprepolymeren herstellen lassen oder auch Aminogruppen aufweisende Polyester des obengenannten Molekulargewichtsbereichs.

Weitere geeignete gegenüber Isocyanatgruppen reaktive Komponenten sind beispielsweise auch die in EP-A 0 689 556 und EP-A 0 937 110 beschriebenen, z. B. durch Umsetzung epoxidierter Fettsäureester mit aliphatischen oder aromatischen Polyolen unter Epoxidringöffung erhältlichen speziellen Polyole.

Auch Hydroxylgruppen enthaltende Polybutadiene können gegebenenfalls eingesetzt werden.

Darüber hinaus sind als gegenüber Isocyanatgruppen reaktive Komponenten auch Schwefel enthaltende Hydroxyverbindungen geeignet. Beispielhaft seien hier genannt einfache Mercaptoalkohole, wie z. B. 2-Mercaptoethanol, 3-Mercaptopropanol, 1,3-Dimercapto-2-propanol, 2,3-Dimercaptopropanol und Dithioerythritol, Thioetherstrukturen enthaltende Alkohole, wie z. B. Di(2-hydroxyethyl)sulfid, 1,2-Bis(2-hydroxyethylmercapto)ethan, Bis(2-hydroxyethyl)disulfid und 1,4-Dithian-2,5-diol, oder Schwefel enthaltende Diole mit Polyesterurethan-, Polythioesterurethan-, Polyesterthiourethan- oder Polythioesterthiourethanstruktur der in der EP-A 1 640 394 genannten Art.

Als gegenüber Isocyanaten reaktive Verbindungen können die erfindungsgemäßen Zusammensetzungen auch niedermolekulare, hydroxy- und/oder aminofunktionelle Komponenten enthalten, d. h. solche eines Molekulargewichtsbereiches von 60 bis 500 g/mol, vorzugsweise von 62 bis 400 g/mol.

Hierbei handelt es sich beispielsweise um einfache ein- oder mehrwertige Alkohole mit 2 bis 14, vorzugsweise 4 bis 10 Kohlenstoffatomen, wie z. B. 1,2-Ethandiol, 1,2- und 1,3-Propandiol, die isomeren Butandiole, Pentandiole, Hexandiole, Heptandiole und Octandiole, 1,10-Decandiol, 1,2- und 1,4-Cyclohexandiol, 1,4-Cyclohexandimethanol, 4,4'-(1-Methylethyliden)-biscyclohexanol, 1,2,3-Propantriol, 1,1,1-Trimethylolethan, 1,2,6-Hexantriol, 1,1,1-Trimethylolpropan, 2,2-Bis(hydroxymethyl)-1,3-propandiol, Bis-(2-hydroxyethyl)-hydrochinon, 1,2,4- und 1,3,5-Trihydroxycyclohexan oder 1,3,5-Tris(2-hydroxyethyl)-isocyanurat.

Beispiele für geeignete niedermolekulare aminofunktionelle Verbindungen sind aliphatische und cycloaliphatische Amine und Aminoalkohole mit primär und/oder sekundär gebundenen Aminogruppen, wie z. B. Cyclohexylamin, 2-Methyl-1,5-pentandiamin, Diethanolamin, Monoethanolamin, Propylamin, Butylamin, Dibutylamin, Hexylamin, Monoisopropanolamin, Diisopropanolamin, Ethylendiamin, 1,3-Diaminopropan, 1,4-Diaminobutan, Isophorondiamin, Diethylentriamin, Ethanolamin, Aminoethylethanolamin, Diaminocyclohexan, Hexamethylendiamin, Methyliminobispropylamin, Iminobispropylamin, Bis(aminopropyl)piperazin, Aminoethylpiperazin, 1,2-Diaminocyclohexan, Triethylentetramin, Tetraethylenpentamin, 1,8-p-Diaminomenthan, Bis(4-aminocyclohexyl)methan, Bis(4-amino-3-methylcyclohexyl)methan, Bis(4-amino-3,5-dimethylcyclohexyl)methan, Bis(4-amino-2,3,5-trimethylcyclohexyl)methan, 1,1-Bis(4-aminocyclohexyl)propan, 2,2-Bis(4-aminocyclohexyl)propan, 1,1-Bis(4-aminocyclohexyl)ethan, 1,1-Bis(4-aminocyclohexyl)butan, 2,2-Bis(4-aminocyclohexyl)butan, 1,1-Bis(4-amino-3-methylcyclohexyl)ethan, 2,2-Bis(4-amino-3-methylcyclohexyl)propan, 1,1-Bis(4-amino-3,5-dimethylcyclohexyl)ethan, 2,2-Bis(4-amino-3,5-dimethylcyclohexyl)propan, 2,2-Bis(4-amino-3,5-dimethylcyclohexyl)butan, 2,4-Diaminodicyclohexylmethan, 4-Aminocyclohexyl-4-amino-3-methylcyclohexylmethan, 4-Amino-3,5-dimethylcyclohexyl-4-amino-3-methylcyclohexylmethan und 2-(4-Aminocyclohexyl)-2-(4-amino-3-methylcyclohexyl)methan.

Beispiele für aromatische Polyamine, insbesondere Diamine, mit Molekulargewichten unter 500 g/mol, die geeignete gegenüber Isocyanaten reaktive Verbindungen B) darstellen, sind z. B. 1,2- und 1,4-Diaminobenzol, 2,4- und 2,6-Diaminotoluol, 2,4'- und/oder 4,4'-Diaminodiphenylmethan, 1,5-Diaminonaphthalin, 4,4',4"-Triaminotriphenylmethan, 4,4'-Bis-(methylamino)-diphenylmethan oder 1-Methyl-2-methylamino-4-aminobenzol. 1-Methyl-3,5-diethyl-2,4-diaminobenzol, 1-Methyl-3,5-diethyl-2,6-diaminobenzol, 1,3,5-Trimethyl-2,4-diaminobenzol, 1,3,5-Triethyl-2,4-diaminobenzol, 3,5,3',5'-Tetraethyl-4,4'-diaminodiphenylmethan, 3,5,3',5'-Tetraisopropyl-4,4'-diaminodiphenylmethan, 3,5-Diethyl-3',5'-diisopropyl-4,4'-diaminodiphenylmethan, 3,3'-Diethyl-5,5'-diisopropyl-4,4'-diaminodiphenylmethan, 1-Methyl-2,6-diamino-3-isopropylbenzol, flüssige Gemische von Polyphenylpolymethylenpolyaminen, wie sie auf bekannte Weise durch Kondensation von Anilin mit Formaldehyd erhältlich sind, sowie beliebige Mischungen solcher Polyamine. In diesem Zusammenhang seien beispielsweise Mischungen von 1-Methyl-3,5-diethyl-2,4-diaminobenzol mit 1-Methyl-3,5-diethyl-2,6-diaminobenzol in einem Gewichtsverhältnis von 50 : 50 bis 85 : 15, vorzugsweise von 65 : 35 bis 80 : 20 besonders erwähnt.

Die Verwendung niedermolekularer aminofunktioneller Polyether mit Molekulargewichten unter 500 g/mol ist ebenfalls möglich. Hierbei handelt es sich beispielsweise um solche mit primären und/oder sekundären, aromatisch oder aliphatisch gebundenen Aminogruppen, deren Aminogruppen gegebenenfalls über Urethan- oder Estergruppen an die Polyetherketten angebunden sind und die nach bekannten, bereits oben zur Herstellung der höhermolekularen Aminopolyether beschriebenen Verfahren zugänglich sind.

Gegebenenfalls können auch sterisch gehinderte aliphatische Diamine mit zwei sekundär gebundenen Aminogruppen als gegenüber Isocyanatgruppen reaktive Komponenten eingesetzt werden, wie z. B. die aus EP-A 0 403 921 bekannten Umsetzungsprodukte aliphatischer und/oder cycloaliphatischer Diamine mit Maleinsäure- oder Fumarsäureestern oder die beispielsweise in der DE-A 19 701 835 beschriebenen Hydrierungsprodukte aus aliphatischen und/oder cycloaliphatischen Diaminen und Ketonen, wie z. B. Diisopropylketon, zugänglicher Schiffscher Basen.

Neben den genannten Ausgangskomponeten A) und B) können dabei gegebenenfalls Hilfs- und Zusatzmittel C), wie z. B. Katalysatoren, oberflächenaktive Mittel, UV-Stabilisatoren, Antioxidantien, Duftstoffe, Formtrennmittel, Füllstoffe und/ oder Pigmente, mitverwendet werden.

Zur Reaktionsbeschleunigung können beispielsweise übliche aus der Polyurethanchemie bekannte Katalysatoren zum Einsatz kommen. Beispielhaft seien hier genannt tert. Amine, wie z. B. Triethylamin, Tributylamin, Dimethylbenzylamin, Diethylbenzylamin, Pyridin, Methylpyridin, Dicyclohexylmethylamin, Dimethylcyclohexylamin, N,N,N',N'-Tetramethyldiaminodiethylether, Bis-(dimethylaminopropyl)-harnstoff, N-Methyl- bzw. N-Ethylmorpholin, N-Cocomorpholin, N-Cyclohexylmorpholin, N,N,N',N'-Tetramethylethylendiamin, N,N,N',N'-Tetramethy-1,3-butandiamin, N,N,N',N'-Tetramethyl-1,6-hexandiamin, Pentamethyldiethylentriamin, N-Methylpiperidin, N-Dimethylaminoethylpiperidin, N,N'-Dimethylpiperazin, N-Methyl-N'-dimethylaminopiperazin, 1,8-Diazabicyclo(5.4.0)undecen-7 (DBU), 1,2-Dimethylimidazol, 2-Methylimidazol, N,N-Dimethylimidazol-β-phenylethylamin, 1,4-Diazabicyclo-(2,2,2)-octan, Bis-(N,N-dimethylaminoethyl)adipat; Alkanolaminverbindungen, wie z. B. Triethanolamin, Triisopropanolamin, N-Methyl- und N-Ethyl-diethanolamin, Dimethylaminoethanol, 2-(N,N-Dimethylaminoethoxy)ethanol, N,N',N"-Tris-(dialkylaminoalkyl)hexahydrotriazine, z.B. N, N',N"-Tris-(dimethylaminopropyl)-s-hexahydrotriazin und/oder Bis(dimethylaminoethyl)ether; Metallsalze, wie z. B. anorganische und/oder organische Verbindungen des Eisens, Bleis, Wismuths, Zinks, und/oder Zinns in üblichen Oxidationsstufen des Metalls, beispielsweise Eisen(II)-chlorid, Eisen(III)-chlorid, Wismut(III)- Wismut(III)-2-ethylhexanoat, Wismut(III)-octoat, Wismut(III)-neodecanoat, Zinkchlorid, Zink-2-ethylcaproat, Zinn(II)-octoat, Zinn(II)-ethylcaproat, Zinn(II)-palmitat, Dibutylzinn(IV)-dilaurat (DBTL), Dibutylzinn(IV)-dichlorid, Dimethylzinn(IV)-dichlorid oder Bleioctoat; Amidine, wie z. B. 2,3-Dimethyl-3,4,5,6-tetrahydropyrimidin; Tetraalkylammoniumhydroxide, wie z. B. Tetramethylammoniumhydroxid; Alkalihydroxide, wie z. B. Natriumhydroxid und Alkalialkoholate, wie z. B. Natriummethylat und Kaliumisopropylat, sowie Alkalisalze von langkettigen Fettsäuren mit 10 bis 20 C-Atomen und gegebenenfalls seitenständigen OH-Gruppen.

Bevorzugte einzusetzende Katalysatoren C) sind tertiäre Amine, Bismuth- und Zinnverbindungen der genannten Art.

Die beispielhaft genannten Katalysatoren können bei der Herstellung der erfindungsgemäßen lichtechten Polyurethan-, Polythiourethan- und/oder Polyharnstoffmassen einzeln oder in Form beliebiger Mischungen untereinander eingesetzt werden und kommen dabei gegebenenfalls in Mengen von 0,001 bis 5,0 Gew.-%, bevorzugt 0,002 bis 2 Gew.-%, berechnet als Gesamtmenge an eingesetzten Katalysatoren bezogen auf die Gesamtmenge der verwendeten Ausgangsverbindungen, zum Einsatz.

Nach dem erfindungsgemäßen Verfahren werden vorzugsweise transparente kompakte Formteile mit hohem Brechungsindex hergestellt. Durch Zusatz geeigneter Treibmittel lassen sich, wenn gewünscht, aber auch geschäumte Formkörper erhalten. Hierfür geeignete Treibmittel sind beispielsweise leicht flüchtige organische Substanzen, wie z.B. Aceton, Ethylacetat, halogensubstituierte Alkane, wie Methylenchlorid, Chloroform, Ethylidenchlorid, Vinylidenchlorid, Monofluortrichlormethan, Chlortrifluormethan oder Dichlordifluormethan, Butan, Hexan, Heptan oder Diethylether und/oder gelöste inerte Gase, wie z.B. Stickstoff, Luft oder Kohlendioxid.

Als chemische Treibmittel C), d. h. Treibmittel die aufgrund einer Reaktion, beispielsweise mit Isocyanatgruppen, gasförmige Produkte bilden, kommen beispielsweise Wasser, Hydratwasser enthaltende Verbindungen, Carbonsäuren, tert.-Alkohole, z. B. t-Butanol, Carbamate, beispielsweise die in der EP-A 1 000 955, insbesondere auf den Seiten 2, Zeilen 5 bis 31 sowie Seite 3, Zeilen 21 bis 42 beschriebenen Carbamate, Carbonate, z. B. Ammoniumcarbonat und/oder Ammoniumhydrogencarbonat und/oder Guanidincarbamat in Betracht.

Eine Treibwirkung kann auch durch Zusatz von bei Temperaturen über Raumtemperatur unter Abspaltung von Gasen, beispielsweise von Stickstoff, sich zersetzenden Verbindungen, z. B. Azoverbindungen wie erzielt werden. Weitere Beispiele für Treibmittel sowie Einzelheiten über die Verwendung von Treibmitteln sind im Kunststoff-Handbuch, Band VII, herausgegeben von Vieweg und Höchtlen, Carl-Hanser-Verlag, München 1966, z.B. auf den Seiten 108 und 109, 453 bis 455 und 507 bis 510 beschrieben.

Erfindungsgemäß können auch oberflächenaktive Zusatzstoffe C) als Emulgatoren und Schaumstabilisatoren mitverwendet werden. Geeignete Emulgatoren sind beispielsweise die Natriumsalze von Ricinusölsulfonaten oder Fettsäuren, Salze von Fettsäuren mit Aminen, wie z. B. ölsaures Diethylamin oder stearinsaures Diethanolamin. Auch Alkali- oder Ammoniumsalze von Sulfonsäuren, wie z. B. von Dodecylbenzolsulfonsäuren, Fettsäuren, wie z. B. Ricinolsäure, oder polymeren Fettsäuren, oder ethoxyliertes Nonylphenol können als oberflächenaktive Zusatzstoffe mitverwendet werden.

Die vorstehend genannten, beim erfindungsgemäßen Verfahren gegebenenfalls mitzuverwendenden Emulgatoren und Stabilisatoren können sowohl einzeln als auch in beliebigen Kombinationen untereinander zum Einsatz kommen.

Die aus den erfindungsgemäß herstellbaren bzw. verwendbaren Polyurethanmassen erhaltenen Körper zeichnen sich bereits als solche, d. h. ohne Zusatz entsprechender Stabilisatoren, durch eine sehr gute Lichtbeständigkeit aus. Dennoch können bei ihrer Herstellung gegebenenfalls UV-Schutzmittel (Lichtstabilisatoren) oder Antioxidantien der bekannten Art als weitere Hilfs- und Zusatzmittel C) mitverwendet werden.

Geeignete UV-Stabilisatoren C) sind beispielsweise Piperidinderivate, wie z. B. 4-Benzoyloxy-2,2,6,6-tetramethylpiperidin, 4-Benzoyloxy-1,2,2,6,6-pentamethylpiperidin, Bis-(2,2,6,6-tetramethyl-4-piperidyl)sebacat, Bis(1,2,2,6,6-pentamethyl-4-piperidyl)sebacat, Methyl(1 ,2,2,6,6-pentamethyl-4-piperidyl)sebacat, Bis-(2,2,6,6-tetramethyl-4-piperidyl)-suberat oder Bis-(2,2,6,6-tetramethyl-4-piperidyl)-dodecandioat, Benzophenonderivate, wie z. B. 2,4-Dihydroxy-, 2-Hydroxy-4-methoxy-, 2-Hydroxy-4-octoxy-, 2-Hydroxy-4-dodecyloxy- oder 2,2'-Dihydroxy-4-dodecyloxy-benzophenon, Benztriazolderivate, wie z. B. 2-(5-Methyl-2-hydroxyphenyl)benztriazol, 2-(5-tert.-Butyl-2-hydroxyphenyl)benztriazol, 2-(5-tert.-Octyl-2-hydroxyphenyl)benztriazol, 2-(5-Dodecyl-2-hydroxyphenyl)benztriazol, 2-(3,5-Di-tert.-butyl-2-hydroxyphenyl)-5-chlorbenztriazol, 2-(3,5-Di-tert.-amyl-2-hydroxyphenyl)benztriazol, 2-(3,5-Di-tert.-butyl-2-hydroxyphenyl)benztriazol, 2-(3-tert.-Butyl-5-methyl-2-hydroxyphenyl)-5-chlorbenztriazol und Veresterungsprodukte von 2-(3-tert.-Butyl-5-propionsäure-2-hydroxyphenyl)benztriazol mit Polyethylenglykol 300, Oxalanilide, wie z. B. 2-Ethyl-2'-ethoxy- oder 4-Methyl-4'-methoxyoxalanilid, Salicylsäureester, wie z. B. Salicylsäurephenylester, Salicylsäure-4-tert.-butylphenylester und Salicylsäure-4-tert.-octylphenylester, Zimtsäureesterderivate, wie z. B. α-Cyano-β-methyl-4-methoxyzimtsäuremethylester, α-Cyano-β-methyl-4-methoxyzimtsäurebutylester, α-Cyano-β-phenylzimtsäureethylester und α-Cyano-β-phenylzimtsäureisooctylester, oder Malonesterderivate, wie z. B. 4-Methoxy-benzylidenmalonsäuredimethylester, 4-Methoxybenzylidenmalonsäurediethylester und 4-Butoxybenzylidenmalonsäuredimethylester. Diese Lichtstabilisatoren können sowohl einzeln als auch in beliebigen Kombinationen untereinander zum Einsatz kommen.

Geeignete Antioxidantien C) sind beispielsweise die bekannten sterisch gehinderten Phenole, wie z. B. 2,6-Di-tert-butyl-4-methylphenol (Ionol), Pentaerythrit-tetrakis(3-(3,5-di-tert-butyl-4-hydroxyphenyl)propionat), Octadecyl-3-(3,5-di-tert-butyl-4-hydroxyphenyl)-propionat, Triethylenglykol-bis(3-tert-butyl-4-hydroxy-5-methylphenyl)propionat, 2,2'-Thio-bis(4-methyl-6-tert-butylphenol), 2,2'-Thiodiethyl-bis[3-(3,5-di-tert-butyl-4-hydroxyphenyl)propionat), die sowohl einzeln als auch in beliebigen Kombinationen untereinander zum Einsatz kommen.

Weitere, gegebenenfalls mitzuverwendende Hilfs- und Zusatzmittel C) sind beispielsweise die bekannten Flammschutzmittel, wie z.B. Tris-chlorethylphosphat, Ammoniumphosphat oder -polyphosphat, Füllstoffe, wie z. B. Bariumsulfat, Kieselgur, Russ, Schlämmkreide oder auch verstärkend wirkende Glasfasern.

Schließlich können gegebenenfalls auch die an sich bekannten inneren Formtrennmittel Farbstoffe, Pigmente, Hydrolyseschutzmittel, fungistatische und bakteriostatisch wirkende Substanzen mitverwendet werden.

Besonders bevorzugt enthält die erfindungsgemäße Zusammensetzung als Komponente C) zumindest ein Formtrennmittel, das ausgewählt ist aus Mono- und / oder Dialkylphosphaten und / oder Mono- und / oder Dialkoxyalkylphosphaten. Als Mono- und / oder Dialkylphosphate sind insbesondere Mono- und / oder Dialkylphosphate mit 2 bis 18 Kohlenstoffatomen im Alkylrest, bevorzugt 8 bis 12 Kohlenstoffatomen. Besonders bevorzugte Mono- beziehungsweise Dialkoxyalkylphosphate besitzen 2 bis 12 Kohlenstoffatome im Alkoxyalkylrest und bis zu drei Ethergruppen pro Alkoxyalkylrest, wobei die vorgenannten Mono- beziehungsweise Alkoxyalkylphosphate insbesondere 4 bis 10 Kohlenstoffatome im Alkoxyalkylrest aufweisen. Die bevorzugten Mono- / Dialkylphosphate und Mono- / Dialkoxyalkylphosphate sind besonders vorteilhaft, da sie neben ihrer eigentlichen Funktion als Formtrennmittel zusätzlich noch einen günstigen Einfluss auf die Reaktionsgeschwindigkeit bei der Umsetzung der Polyisocyanatkomponente mit der Thiolkomponente insofern zeigen, dass durch deren Zusatz die Reaktionsgeschwindigkeit der beiden Komponenten miteinander reduziert wird. Dies führt letztlich zu optisch hochwertigeren Formkörpern und einer einfacheren Handhabung der Zusammensetzung nach dem Vermischen der Komponenten.

Geeignete interne Entformungsmittel sind beispielsweise Methylphosphat, Dimethylphosphat, Methoxyethylphosphat, Methoxypropylphosphat, Di-(methoxyethyl)phosphat, Methoxyethyl-ethoxyethylphosphat, Methoxyethylpropoxyethylphosphat, Di-(methoxypropyl)phosphat, Ethylphosphat, Diethylphosphat, Ethoxyethylphosphat, Di-(ethoxyethyl)phosphat, Ethoxypropylphosphat, Ethoxyethylpropoxyethylphosphat, Di-(ethoxypropyl)phosphat, Ethoxyethyl-butoxyethylphosphat, Isopropylphosphat, Diisopropylphosphat, Propoxyethylphosphat, Di-(propoxyethyl)phosphat, Propoxypropylphosphat, Di-(propoxylpropyl)phosphate, Butylphosphat, Dibutylphosphat, Butoxyethylphosphat, Butoxypropylphosphat, Di-(butoxyethyl) phosphat, Pentoxyethylphosphat, Bis-(2-ethylhexyl)phosphat, Di-(hexyloxyethyl)phosphat, Octylphosphat, Dioctylphosphat, Decylphosphat, Isodecylphosphat, Diisodecylphosphat, Isodecyloxyethylphosphat, Di-(decyloxyethyl)phosphat, Dodecylphosphat, Didoceylphosphat, Tridecanolephosphat, Bis(tridecanol)-phosphat, Stearylphospat, Distearylphosphat und beliebige Gemische solcher Verbindungen.

Zweckmäßigerweise enthalten die erfindungsgemäßen Zusammensetzungen bezogen auf die Gesamtzusammensetzung 0,002 bis 4 Gew.-% an Mono- / Dialkylphosphaten und / oder Mono- / Dialkylalkoxyphosphaten, bevorzugt 0,005 bis 2 Gew.-%. Die vorgenannten Einsatzmengen beziehen sich dabei auf den Gesamtgehalt dieser Substanzen als Formtrennmittel.

Eine besonders bevorzugte erfindungsgemäße Zusammensetzung zur Herstellung transparenter Polythiourethan-Körper oder transparenten Polythiourethans enthält oder besteht aus
A) 35 bis 65 Gew.-% bezogen auf die Zusammensetzung, insbesondere 45 bis 55 Gew.-% einer Polyisocyanatkomponente enthaltend zumindest 70 bis 99,8 Gew.-%, insbesondere 85 bis 99,7 Gew.-% bezogen auf die Polyisocyanatkomponente an über Gasphasenphosgenierung gemäß der vorliegenden Erfindung erzeugtes 1,3- und/oder 1,4- Xylylendiisocyanat (XDI),
B) 35 bis 65 Gew.-% bezogen auf die Zusammensetzung, insbesondere 45 bis 55 Gew.-% einer Thiolkomponente enthaltend zumindest ein Polythiol mit einer Funktionalität an Thiolgruppen von wenigstens 2 pro Molekül, wobei die Thiolkomponente insbesondere aus einem Polythiol mit einer Funktionalität an Thiolgruppen von 3 pro Molekül, nämlich DMPT (4-Mercaptomethyl-1,8-dimercapto-3,6-dithiaoctan), besteht,
   sowie
C) Hilfs- und Zusatzmittel umfassend 0,002 bis 4 Gew.-%, insbesondere 0,005 bis 2 Gew.-% eines Formtrennmittels ausgewählt aus Mono- und/ oder Dialkylphosphaten mit 2 bis 18 Kohlenstoffatomen im Alkylrest und/oder Mono- und/ oder Dialkoxyalkylphosphaten mit 2 bis 12 Kohlenstoffatomen im Alkoxyalkylrest und bis zu drei Ethergruppen pro Alkoxyalkylrest,
   wobei das Verhältnis von Isocyanatgruppen zu gegenüber Isocyanaten reaktiven Gruppen 0,5 : 1 bis 2,0 : 1 beträgt.

Eine besonders bevorzugte erfindungsgemäße Zusammensetzung zur Herstellung eines transparenten Polyurethans oder Polyurethan-Körpers enthält oder besteht aus
A) 60 bis 97 Gew.-% bezogen auf die Zusammensetzung, insbesondere 80 bis 95 Gew.-% einer Polyisocyanatkomponente enthaltend zumindest 70 bis 99,8 Gew.-%, insbesondere 85 bis 99,7 Gew.-% bezogen auf die Polyisocyanatkomponente an über Gasphasenphosgenierung gemäß der vorliegenden Erfindung erzeugtes 1,3- und/oder 1,4- Xylylendiisocyanat (XDI),
B) 3 bis 40 Gew.-% bezogen auf die Zusammensetzung, insbesondere 5 bis 10 Gew.-% einer Polyolkomponente enthaltend oder bestehend aus zumindest ein Polyol mit einer Funktionalität an Hydroxylgruppen von wenigstens 2 pro Molekül, sowie
C) Hilfs- und Zusatzmittel umfassend 0,002 bis 4 Gew.-%, insbesondere 0,005 bis 2 Gew.-% eines Formtrennmittels ausgewählt aus Mono- und/ oder Dialkylphosphaten mit 2 bis 18 Kohlenstoffatomen im Alkylrest und/oder Mono- und/ oder Dialkoxyalkylphosphaten mit 2 bis 12 Kohlenstoffatomen im Alkoxyalkylrest und bis zu drei Ethergruppen pro Alkoxyalkylrest, bevorzugt Dibutylphosphat,
wobei das Verhältnis von Isocyanatgruppen zu gegenüber Isocyanaten reaktiven Gruppen 0,5 : 1 bis 15,0 : 1 beträgt, bevorzugt 0,5 : 1 bis 2,0 :1.

Ein weiterer Gegenstand der vorliegenden Erfindung betrifft ein Verfahren zur Herstellung transparenter Polythiourethankörper durch Umsetzung einer Zusammensetzung enthaltend oder bestehend aus
A) einer Polyisocyanatkomponente enthaltend über Gasphasenphosgenierung gemäß der vorliegenden Erfindung hergestelltes oder herstellbares 1,3- und/oder 1,4-Xylylendiisocyanat (XDI) sowie gewünschtenfalls wenigstens ein weiteres Polyisocyanat mit einer Funktionalität an Isocyanatgruppen von wenigstens 2 pro Molekül,
B) einer Thiolkomponente enthaltend oder bestehend aus zumindest ein Polythiol mit einer Funktionalität an Thiolgruppen von wenigstens 2 pro Molekül,
   sowie gegebenenfalls
C) weitere Hilfs- und Zusatzmittel,
wobei das Verhältnis von Isocyanatgruppen zu gegenüber Isocyanaten reaktiven Gruppen 0,5 : 1 bis 2,0 : 1 beträgt.

Ein weiterer Gegenstand der vorliegenden Erfindung betrifft ein Verfahren zur Herstellung transparenter Polyurethane durch Umsetzung einer Zusammensetzung enthaltend oder bestehend aus
A) einer Polyisocyanatkomponente enthaltend über Gasphasenphosgenierung gemäß der vorliegenden Erfindung hergestelltes oder herstellbares 1,3- und/oder 1,4-Xylylendiisocyanat (XDI), sowie gewünschtenfalls wenigstens ein weiteres Polyisocyanat mit einer Funktionalität an Isocyanatgruppen von wenigstens 2 pro Molekül,
B) einer Polyolkomponente enthaltend oder bestehend aus zumindest ein Polyol mit einer Funktionalität an Hydroxylgruppen von wenigstens 2 pro Molekül,
   sowie gegebenenfalls
C) weitere Hilfs- und Zusatzmittel,
wobei das Verhältnis von Isocyanatgruppen zu gegenüber Isocyanaten reaktiven Gruppen 0,5 : 1 bis 15,0 : 1 beträgt. Unabhängig von der Art der gewählten Ausgangsstoffe erfolgt beim erfindungsgemäßen Verfahren die Umsetzung der Polyisocyanatgemische A) mit der Thiolkomponente und/ oder der Polyolkomponente sowie gegebenenfalls weiteren gegenüber Isocyanatgruppen reaktiven Komponenten unter Einhaltung eines Äquivalentverhältnisses von Isocyanatgruppen zu gegenüber Isocyanaten reaktiven Gruppen von 0,5 : 1 bis 15,0 : 1, vorzugsweise von 0,7 : 1 bis 2,0 : 1, besonders bevorzugt von 0,8 : 1 bis 1,2 : 1.

Bei dem erfindungsgemäßen Verfahren werden die Komponenten der erfindungsgemäßen Zusammensetzung bevorzugt, gegebenenfalls in lösemittelfreier Form, im oben angegebenen Äquivalentverhältnis von Isocyanatgruppen zu gegenüber Isocyanaten reaktiven Gruppen, mit Hilfe geeigneter Mischaggregate gemischt und nach beliebigen Methoden in offene oder geschlossene Formen, beispielsweise durch einfaches Vergießen von Hand, bevorzugt aber mit Hilfe geeigneter Maschinen, wie z. B. den in der Polyurethantechnologie üblichen Niederdruck- oder Hochdruckmaschinen oder nach dem RIM-Verfahren, eingefüllt. Die Aushärtung kann in einem Temperaturbereich von 40 bis 180°C, vorzugsweise von 50 bis 140°C, besonders bevorzugt von 60 bis 120°C, und gegebenenfalls unter einem erhöhten Druck von bis zu 300 bar, vorzugsweise bis 100 bar, besonders bevorzugt bis zu 40 bar durchgeführt werden.

Dabei werden die Polyisocyanate und gegebenenfalls auch die anderen Ausgangskomponenten gegebenenfalls durch Anlegen von Vakuum entgast.

In der Regel können die so aus den erfindungsgemäß hergestellten Formkörper nach kurzer Zeit entformt werden, beispielsweise nach 2 bis 60 min. Gegebenenfalls kann sich eine Nachhärtung bei einer Temperatur von 50 bis 100 °C anschließen, vorzugsweise bei 60 bis 90°C.

Auf diese Weise erhält man kompakte licht- und wetterbeständige Polythiourethankörper, die eine hohe Beständigkeit gegenüber Lösungsmitteln und Chemikalien sowie hervorragende mechanische Eigenschaften, insbesondere eine exzellente Wärmeformbeständigkeit auch bei höheren Temperaturen von beispielsweise 80°C, besitzen. Gegenüber den bisher bekannten Systemen, die unter Verwendung von über Flüssigphasenphosgenierung erzeugten Diisocyanaten hergestellt wurden, zeichnen sich die erfindungsgemäßen Formkörper durch eine höhere Transparenz und geringere Trübung aus.

Ein weiterer Gegenstand der vorliegenden Erfindung betrifft einen kompakten transparenten Polythiourethan-Körper oder Polyurethan-Körper, erhältlich durch Umsetzung der Komponenten der erfindungsgemäßen Zusammensetzung. Wie bereits vorstehend ausgeführt wurde, ist ein so erzeugter kompakter transparenter Polythiourethan-Körper wegen seiner guten optischen Eigenschaften besonders bevorzugt. Bei dem Polythiourethan-Körper oder dem Polyurethan-Körper kann es sich hierbei um ein Glasersatzteil, ein optisches, optoelektronisches oder elektronisches Bauteil, um eine optische Linse oder um ein Brillenglas handeln. Konkrete Anwendungen sind beispielsweise die Herstellung von bzw. die Verwendung als Glasersatz-Scheiben, wie z. B. Sonnendächern, Front-, Heck- oder Seitenscheiben im Fahrzeug- oder Flugzeugbau, als Sicherheitsglas, Solarmodulen, Leuchtdioden, Linsen oder Kollimatoren, wie sie beispielsweise als Vorsatzoptik in LED-Leuchten oder Automobilscheinwerfern zum Einsatz kommen.

Besonders bevorzugtes Einsatzgebiet für die aus den erfindungsgemäßen Zusammensetzungen erhältlichen erfindungsgemäßen Polythiourethan-Formkörpern ist aber die Herstellung leichtgewichtiger Kunststoff-Brillengläser mit hohem Brechungsindex und hoher Abbe-Zahl. Erfindungsgemäß hergestellte Brillengläser zeichnen sich durch hervorragende mechanische Eigenschaften, insbesondere Härte und Schlagfestigkeit sowie gute Kratzfestigkeit aus und sind darüberhinaus leicht zu bearbeiten und beliebig einfärbbar.

Die Erfindung wird im Folgenden anhand von Ausführungsbeispielen und einer Abbildung (Abb. 1) näher erörtert.

### Experimenteller Teil und Beispiele:

### Thermische Stabilität von reinem 1,3-XDA

Durch Temperversuche wurde die thermische Zersetzung von 1,3-XDA im infrage kommenden Siedebereich von 220-240°C untersucht. Hierzu wurde 1,3-XDA unter Stickstoffatmosphäre bei der jeweiligen Temperatur 8 h getempert, wobei zu Beginn und dann stündlich Proben zur Bestimmung des nicht flüchtigen Anteils durch GC-Analyse mit internem Standard gezogen wurden.

**GC-Methode zur Bestimmung von 1,3-XDA mit internem Standard**

| | |
|---|---|
| Herstellung der Standardlösung: | 1,4 g Anthracen werden in einem Messkolben bei 23°C in 1 I Ethylacetat gelöst |
| Probenvorbereitung: | ca. 1-2 g der Probe werden mit genau 20 ml Standardlösung versetzt und homogenisiert |
| Gas-Chromatograph: | Hewlett Packard, HP 5890, Serie B |
| Trennsäule: | ZB 65 HT (Fa. Zebron), fused Silica, Länge 30 m, innerer Durchmesser 0,32 mm, Filmdicke 0,25 µm |
| Temperaturen: | Injektor 250°C, Detektor (FID) 350°C, Ofen: Start 80°C, Haltezeit 0 min , Heizrate 10°K/min auf 350°C, Haltezeit 10 min |
| Trägergas: | Helium, Säulendruck 1,0 - 1,1 bar abs., Splitausgang 100 ml/h, Septumspülung 5-1 ml/h |

Die Ergebnisse sind in Tabelle 1 zusammen gestellt.

**Legende:**

| | |
|---|---|
| Niedrigsieder: | Flächenprozent (FI.%) niedrig siedende Komponente mit Retentionszeit 5,5 min. |
| 1,3- XDA: | FI.% an 1,3-XDA mit Retentionszeit 9,5 min. |
| Nicht flüchtig (Polyamine): | 100% - (FI.% Niedrigsieder + FI.% 1,3- XDA) |

**Tabelle 1:**

| | 220°C | | | 230°C | | | 240°C | | |
|---|---|---|---|---|---|---|---|---|---|
| Komponente | Niedrigsieder | 1,3-XDA | nicht flüchtig | Niedripsieder | 1,3-XDA | nicht flüchtig | Niedrigsieder | 1,3-XDA | nicht flüchtig |
| 0 h | 0,01% | 99,29% | 0,70% | 0,08% | 98,01% | 1,91% | 0,11% | 98,74% | 1,15% |
| 1 h | 0,15% | 97,52% | 2,33% | 0,32% | 94,39% | 5,29% | 0,29% | 96,18% | 3,53% |
| 2 h | 0,31% | 95,90% | 3,79% | 0,70% | 90,92% | 8,38% | 2,06% | 91,61% | 6,33% |
| 3 h | 0,72% | 90,43% | 8,85% | 1,29% | 88,41 % | 10,30% | 5,40% | 79,96% | 14,64% |
| 4 h | 2,01% | 88,22% | 9,77% | 3,12% | 83,85% | 13,03% | 10,60% | 70,21% | 19,20% |
| 5 h | 3,59% | 83,40% | 13,01% | 5,08% | 78,01% | 16,91% | 14,45% | 50,13% | 35,42% |
| 6 h | 5,05% | 74,64% | 20,31% | 7,92% | 68,51% | 23,57% | 19,78% | 33,42% | 46,81% |
| 7 h | 7,56% | 66,93% | 25,51% | 11,09% | 53,14% | 35,77% | 22,04% | 16,59% | 61,37% |
| 8 h | 11,45% | 54,91% | 33,64% | 15,49% | 33,64% | 50,87% | 7,48% | 0,00% | 92,52% |

Die Ergebnisse der Temperversuche zeigen, dass 1,3- XDA sich im für die Gasphasenphosgenierung technisch relevanten Siedebereich von 220-240°C recht schnell unter Bildung von nichtflüchtigen Polyaminen zersetzt.

### Thermische Stabilität von stabilisiertem 1,3-XDA

Durch Temperversuche wurde die thermische Zersetzung von 1,3-XDA nach Zusatz von verschiedenen Stabilisatoren untersucht. Hierzu wurde 1,3-XDA nach Zusatz von 500 ppm des jeweiligen Stabilisators unter Stickstoffatmosphäre 8 h bei 230°C getempert, wobei zu Beginn und dann stündlich Proben für die GC-Analyse mit internem Standard gezogen wurden. Der Anteil nicht flüchtiger Polyamine wurde wie oben angeben bestimmt und ist in Tabelle 2 zusammen gestellt.

| | |
|---|---|
| Stabilisatoren: Ionol: | 2,6-Di-tert.butyl-p-kresol |
| | DBSS: Dodecylbenzosulfonsäure |
| | Tinuvin 571: Alkylphenol-substituiertes Benztriazol (BASF) |
| | p-TSA: p-Toluolsulfonsäureamid |

**Tabelle 2:**

| Stabilisator | ohne | Ionol | DBSS | Tinuvin 571 | P-TSA |
|---|---|---|---|---|---|
| 0 h | 0,61% | 0,48% | 0,51% | 0,31% | 0,43% |
| 1 h | 5,29% | 0,50% | 0,59% | 0,41% | 1,07% |
| 2 h | 8,38% | 1,23% | 1,30% | 0,65% | 1,13% |
| 3 h | 10,30% | 1,58% | 1,62% | 0,73% | 1,38% |
| 4 h | 13,03% | 2,01% | 2,22% | 0,83% | 1,92% |
| 5 h | 16,91% | 2,53% | 2,73% | 0,98% | 3,65% |
| 6 h | 23,57% | 3,51% | 3,71% | 1,13% | 3,57% |
| 7 h | 35,77% | 4,23% | 4,34% | 1,28% | 4,80% |
| 8 h | 50,87% | 5,27% | 5,45% | 1,43% | 3,43% |

Die Ergebnisse zeigen, dass die thermische Stabilität von 1,3-XDA im für die Gasphasenphosgenierung technisch relevanten Siedebereich bei 230°C durch Zusatz von Stabilisatoren deutlich verbessert werden kann.

### Durchführung der Phosgenierung von 1,3-XDA

### Beispiel 1 (erfindungsgemäß):

In einer Anlage zur Gasphasenphosgenierung mit einer Aminverdampfungsstufe entsprechend Abb. 1, einem Rohreaktor (L: 9350 mm, Innendurchmesser 134,5 mm) mit einer auf der Reaktorachse angeordneten Koaxialdüse (Innendurchmesser 134,5 mm) und einer nachgeschalteten Isocyanatkondensationsstufe werden kontinuierlich 200 kg/h 1,3-XDA bei einem Druck von 650 mbar abs. unter Einleitung eines Stickstoffstroms von 10 kg/h verdampft, wobei die Temperatur im Umpumpkreislauf (3200 kg/h) durch Kühlung in einem Wärmetauscher (WT) bei 150°C gehalten wird. Dem Umpumpkreislauf wurden vorher 500 ppm Tinuvin ® 571 zugesetzt. Die Vorlauftemperatur zum Verdampfer (V) beträgt 255°C, die Eintrittstemperatur des Kühlmediums in den Wärmetauscher (WT) 40°C und die mittlere Verweilzeit des 1,3-XDA im Umpumpkreislauf 35 Minuten. Der Strom aus gasförmigem 1,3-XDA und Stickstoff wird nach Verlassen des Verdampfers in einem weiteren Wärmetauscher auf 280°C erhitzt und über die Koaxialdüse dem Reaktor zugeführt. Gleichzeitig werden parallel dazu 750 kg/h Phosgen auf 310°C erhitzt und auf dem von der Düse freigelassenen Ringraum ebenfalls dem Reaktor kontinuierlich zugeführt, in welchem die beiden Eduktströme gemischt und zur Reaktion gebracht werden. Dabei beträgt die Geschwindigkeit des Gasstroms im Reaktor ca. 20 m/s und das Geschwindigkeitsverhältnis von Amin-/Stickstoff- zu Phosgenstrom 8,8 . Der Druck an der Vakuumpumpe beträgt 600 mbar abs. Nach einer mittleren Verweilzeit im Reaktor von 0,48 s wird der das Reaktionsprodukt 1,3-XDI enthaltende Gasstrom durch Einspritzkühlung mit Monochlorbenzol abgekühlt und kondensiert, wobei die Temperatur der flüssigen Phase in der Quenche ca. 90°C beträgt. Der gaschromatographisch bestimmte Gehalt an 3-Chlormethyl-benzylisocyanat beträgt 0,4 % bezogen auf die Summe aus 1,3-XDI und 3-Cl-XI. Nachfolgend wird das Reaktionsgemisch von HCl und Phosgen befreit und destillativ aufgearbeitet. Die Ausbeute an 1,3-XDI beträgt 95% der Theorie.

### Beispiel 2 (erfindungsgemäß):

In der oben beschriebenen Anlage werden analog 160 kg/h 1,3-XDA bei einem Druck von 500 mbar abs. unter Einleitung eines Stickstoffstroms von 4 kg/h verdampft, wobei die Temperatur im Umpumpkreislauf (3200 kg/h) durch Kühlung in einem Wärmetauscher (WT) bei 150°C gehalten wird. Dem Umpumpkreislauf wurden vorher 500 ppm Tinuvin ® 571 zugesetzt. Die Vorlauftemperatur zum Verdampfer (V) beträgt 240°C, die Eintrittstemperatur des Kühlmediums in den Wärmetauscher (WT) 40°C und die mittlere Verweilzeit des 1,3-XDA im Umpumpkreislauf 43 Minuten. Der Strom aus gasförmigem1,3-XDA und Stickstoff wird in einem weiteren Wärmetauscher auf 280°C erhitzt und über die Koaxialdüse dem Reaktor zugeführt. Gleichzeitig werden parallel dazu 500 kg/h Phosgen auf 310°C erhitzt und auf dem von der Düse freigelassenen Ringraum ebenfalls dem Reaktor kontinuierlich zugeführt, in welchem die beiden Eduktströme gemischt und zur Reaktion gebracht werden. Dabei beträgt die Geschwindigkeit des Gasstroms im Reaktor ca. 20 m/s und das Geschwindigkeitsverhältnis von Amin- zu Phosgenstrom 8,8. Nach einer mittleren Verweilzeit im Reaktor von 0,46 s wird der das Reaktionsprodukt 1,3-XDI enthaltende Gasstrom durch Einspritzkühlung mit Monochlorbenzol abgekühlt und kondensiert, wobei die Temperatur der flüssigen Phase in der Quenche ca. 90°C beträgt. Der gaschromatographisch bestimmte Gehalt an 3-Chlormethyl-benzylisocyanat beträgt 0,3 % bezogen auf die Summe aus 1,3-XDI und 1,3-Cl-XI. Nachfolgend wir das Reaktionsgemisch von HCl und Phosgen befreit und destillativ aufgearbeitet. Die Ausbeute an 1,3-XDI beträgt 92% der Theorie.

Herstellung von über Flüssigphasen-Phosgenierung erzeugtem XDI zum Vergleich:

### Beispiel 3 (Vergleich):

Unter Rühren und Kühlen wird eine Lösung von 5 Gew.-Teilen 1,3-XDA in 50 Gew.-Teilen Monochlorbenzol bei 0 - 10°C zu einer Lösung von 20 Gew.-Teilen Phosgen in 25 Gew.-Teilen Monochlorbenzol dosiert und nach beendeter Zugabe auf Raumtemperatur kommen gelassen. Anschließend wird die Temperatur unter Einleiten von Phosgen entsprechend der Gasentwicklung bis zum Rückfluss erhöht und solange weiter phosgeniert, bis die Lösung allmählich klar wird. Sobald der Klarpunkt erreicht ist (ca. 4-5 h), wird noch 30 Minuten nachphosgeniert. Dann wird die Phosgeneinleitung beendet und unter Einleitung von Stickstoff solange am Rückfluss gekocht, bis im Abgas kein Phosgen mehr nachweisbar ist.

Der gaschromatographisch bestimmte Gehalt an 3-Chlormethyl-benzylisocyanat beträgt 0,9 % bezogen auf die Summe aus 1,3-XDI und 1,3-Cl-XI. Anschließend wird das Reaktionsgemisch destillativ aufgearbeitet, wobei XDI als farblose Flüssigkeit mit einem Siedepunkt von 130°C / 0,2mbar erhalten wird. Die Ausbeute an 1,3-XDI beträgt ca. 80% der Theorie.

### Beispiel 4 (erfindungsgemäß):

2 kg des in Beispiel 2 erhaltenen XDI werden über eine Kolonne fraktioniert destilliert. Die ersten 500 g werden als Vorlauf verworfen, als Hauptfraktion werden 1 kg farbloses 1,3-XDI erhalten. Diese Hauptfraktion wird mit 1,4 g Zelec UN (Steppan) bei Raumtemperatur versetzt und 24 h stehen gelassen. Der HC Gehalt der Probe gemessen nach ASTM Vorschrift D4663-98 liegt nach der Destillation bei 105 ppm.

### Beispiel 5 (Vergleich):

2 kg des in Beispiel 3 erhaltenen XDI werden über eine Kolonne fraktioniert destilliert. Die ersten 800 g werden als Vorlauf verworfen, als Hauptfraktion werden 700 g farbloses 1,3-XDI erhalten. Diese Hauptfraktion wird mit 0,98 g Zelec UN bei Raumtemperatur versetzt und 24 h stehen gelassen. Der HC Gehalt der Probe gemessen nach ASTM Vorschrift D4663-98 liegt nach der Destillation bei 108 ppm.

Herstellung von Polythiourethan Brillengläsern:

### Beispiel 6 (erfindungsgemäß):

Zunächst wurde eine Gießform erstellt, indem zwei Glasformschalen (85mm Durchmesser, Innenradius 88 mm, Shamir Insight, Inc., IL) im Abstand von 8 mm mit einem Kunststoff Dichtring zusammengeklemmt wurden, so dass eine Gießkavität gebildet wird. Der Formabstand beträgt en jeder Stelle der Linse 8 mm.

Das Gießsystem wurde wie folgt hergestellt:
In einem Kolben wurden in 94,59 g 1,3-XDI aus Beispiel 4 0,002 g Dibutylzinndichlorid (DBC) gelöst und für 30 Minuten bei 10 mbar evakuiert. 90,00 g DMPT (4-Mercaptomethyl-1,8-dimercapto-3,6-dithiaoctan, Bruno Bock GmbH, eingesetzt wie erhalten) wurden anschließend in den Kolben dazugegeben, und die finale Mischung wurde für 30 Minuten bei 10 mbar gerührt und entgast. Diese Mischung wurde dann durch einen 5 µm Filter filtriert, auf eine Spritze aufgezogen und die Gießform damit vollständig gefüllt.

Die gefüllte Gießform wurde im Trockenschrank mit folgendem Temperaturprofil gehärtet: 15 Stunden bei 65 °C; 2 Stunden bei 100 °C und weitere 2 Stunden bei 120 °C getempert. Danach wurde die Gießform auf Raumtemperatur abgekühlt und nach vollständigem Abkühlen zunächst die Manschette und dann die zwei Glaskörper manuell entfernt.

Auf diese Weise wurde ein klarer, transparenter und trübungsfreier Brillenglasrohling erhalten. Die Messung der Brechungsindices und Abbe-Zahlen erfolgte an einem Abbe-Refraktometer Modell AR4D der Firma A.KRÜSS Optronic GmbH bei 23 °C.

Die Transmission betrug bei der Normlichtart D65 90,3 %, der Haze lag bei 2,1. Der Brechungsindex nE betrug 1,67 bei 23 °C. Die Transmissions- und Haze Messungen wurden nach ASTM D 1003 mit einem Haze-Gard Plus der Fa. Byk bei Normlichtart D65 (definiert in DIN 6173) durchgeführt.

### Beispiel 7 (Vergleich):

Analog zu Beispiel 6 wurde ein Brillenglasrohling hergestellt unter Verwendung von 1,3-XDI aus Beispiel 5. Dieser Brillenglasrohling war vollkommen trübe, die Transmission lag bei 29,7 %, der Haze bei 100, eine Messung des Brechungsindex war nicht möglich.

## Patentansprüche

1. Verfahren zur Herstellung von 1,3- und/oder 1,4- Xylylendiisocyanat (XDI) durch Phosgenierung von 1,3- und/oder 1,4- Xylylendiamin (XDA) mit Phosgen in der Gasphase, **dadurch gekennzeichnet, dass** das Xylylendiamin/die Xylylendiamine, unter Zusatz von Stabilisatoren und/oder eines Inertgases,
a) bei < 1.000 mbar verdampft wird/werden, wobei
b) die mittlere Verweilzeit im Verdampferkreislauf 5 bis 90 Minuten beträgt und
c) die Temperatur im Umpumpkreislauf der Verdampfung bei 40 bis 190°C liegt
und
d) die um weitere 10-100°K über die Verdampfungungstemperatur des XDA unter dem gegebenen Druck erhitzten XDA-Brüden nach bekannten Verfahren der Gasphasenphosgenierung mit Phosgen umgesetzt werden.

2. Verfahren gemäß Anspruch 1, wobei das Xylylendiamin/die Xylylendiamine bei 350 bis 900 mbar, verdampft wird/werden.

3. Verfahren gemäß Anspruch 1, wobei das Xylylendiamin/die Xylylendiamine bei 500 bis 800 mbar, verdampft wird/werden.

4. Verfahren gemäß einem der Ansprüche 1 bis 3, wobei die mittlere Verweilzeit im Verdampferkreislauf 10 bis 60 Minuten beträgt.

5. Verfahren gemäß einem der Ansprüche 1 bis 3, wobei die mittlere Verweilzeit im Verdampferkreislauf 10 bis 45 Minuten beträgt.

6. Verfahren gemäß einem der Ansprüche 1 bis 5, wobei die Temperatur im Umpumpkreislauf der Verdampfung 60 bis 170°C beträgt.

7. Verfahren gemäß einem der Ansprüche 1 bis 5, wobei die Temperatur im Umpumpkreislauf der Verdampfung 60 bis 150°C beträgt

8. Verfahren gemäß einem der Ansprüche 1 bis 7, wobei die XDA-Brüden um weitere 20 bis 50 K über die Verdampfungungstemperatur des XDA unter dem gegebenen Druck erhitzt werden, und dann nach bekannten Verfahren der Gasphasenphosgenierung mit Phosgen umgesetzt werden.

9. Verfahren gemäß einem der Ansprüche 1 bis 8, wobei dem XDA vor der Verdampfung ein oder mehrere Stabilisatoren in Konzentrationen von 50-2.000 ppm bezogen auf XDA zugegeben werden.

10. Verfahren gemäß Anspruch 9, wobei es sich bei den Stabilisatoren um sterisch gehinderte Phenole und/oder Alkyl-substituierte Benzosulfonsäuren handelt.

11. Verfahren gemäß einem der Ansprüche 1 bis 10, wobei zwischen der Verdampfung des XDA und der Überhitzung der XDA-Brüden und/oder nach der Überhitzung der XDA-Brüden eine Abscheidung von XDA-Tröpfchen mittels eines Tröpfchenabscheiders erfolgt.

12. Verfahren gemäß Anspruch 11, wobei der/die zur Überhitzung verwendete/n Apparatur/en auch als Tröpfchenabscheider fungiert/fungieren.

13. 1,3- und/oder 1,4- Xylylendiisocyanat hergestellt oder herstellbar nach einem Verfahren gemäß einem der Ansprüche 1 bis 12.

14. Verwendung von 1,3- und/oder 1,4- Xylylendiisocyanat gemäß Anspruch 13 zur Herstellung transparenter Polyurethane oder Polythiourethane, insbesondere zur Herstellung transparenter Polyurethan- oder Polythiourethan-Formkörper.

15. Polyurethan- oder Polythiourethan-Formkörper, hergestellt unter Verwendung von 1,3- und/oder 1,4- Xylylendiisocyanat gemäß Anspruch 13.

## Claims

1. Method for producing 1,3- and/or 1,4-xylylene diisocyanate (XDI) by phosgenation of 1,3- and/or 1,4-xylylenediamine (XDA) with phosgene in the gas phase, **characterized in that** the xylylenediamine/xylylenediamines, with addition of stabilizers and/or an inert gas,
a) is/are vaporized at < 1000 mbar, with
b) the mean residence time in the vaporizer circuit being 5 to 90 minutes and
c) the temperature in the pumping circuit of the vaporization being 40 to 190°C, and
d) the XDA vapors, heated by a further 10-100°K above the vaporization temperature of the XDA under the prevailing pressure, are reacted with phosgene by known methods of gas-phase phosgenation.

2. Method according to Claim 1, the xylylenediamine/xylylenediamines being vaporized at 350 to 900 mbar.

3. Method according to Claim 1, the xylylenediamine/xylylenediamines being vaporized at 500 to 800 mbar.

4. Method according to any of claims 1 to 3, the mean residence time in the vaporizer circuit being 10 to 60 minutes.

5. Method according to any of Claims 1 to 3, the mean residence time in the vaporizer circuit being 10 to 45 minutes.

6. Method according to any of Claims 1 to 5, the temperature in the pumping circuit of the vaporization being 60 to 170°C.

7. Method according to any of Claims 1 to 5, the temperature in the pumping circuit of the vaporization being 60 to 150°C.

8. Method according to any of Claims 1 to 7, the XDA vapors being heated by a further 20 to 50 K above the vaporization temperature of the XDA under the prevailing pressure, and then being reacted with phosgene via known methods of gas-phase phosgenation.

9. Method according to any of Claims 1 to 8, the XDA being admixed prior to the vaporization with one or more stabilizers in concentrations of 50-2000 ppm based on XDA.

10. Method according to Claim 9, the stabilizers being sterically hindered phenols and/or alkyl-substituted benzosulfonic acids.

11. Method according to any of Claims 1 to 10, with separation of XDA droplets by means of a droplet separator between the vaporization of the XDA and the superheating of the XDA vapors and/or after the superheating of the XDA vapors.

12. Method according to Claim 11, the apparatus used for the superheating also functioning as droplet separator(s).

13. 1,3- and/or 1,4-xylylene diisocyanate produced or producible by a method according to any of Claims 1 to 12.

14. Use of 1,3- and/or 1,4-xylylene diisocyanate according to Claim 13 for producing transparent polyurethanes or polythiourethanes, especially for producing transparent polyurethane or polythiourethane moldings.

15. Polyurethane or polythiourethane molding produced using 1,3- and/or 1,4-xylylene diisocyanate according to Claim 13.

## Revendications

1. Procédé de fabrication de diisocyanate de 1,3- et/ou 1,4-xylylène (XDI) par phosgénation de 1,3- et/ou 1,4-xylylène-diamine (XDA) avec du phosgène dans la phase gazeuse, **caractérisé en ce que** la xylylène-diamine/les xylylène-diamines
a) sont évaporées à < 1 000 mbar avec ajout de stabilisateurs et/ou d'un gaz inerte,
b) le temps de séjour moyen dans le circuit de l'évaporateur étant de 5 à 90 minutes et
c) la température dans le circuit de pompage de l'évaporation étant de 40 à 190 °C,
et
d) les vapeurs de XDA portées 10 à 100 °K au-dessus de la température d'évaporation de la XDA à la pression donnée étant mises en réaction avec du phosgène selon le procédé connu de phosgénation en phase gazeuse.

2. Procédé selon la revendication 1, dans lequel la xylylène-diamine/les xylylène-diamines sont évaporées à 350 à 900 mbar.

3. Procédé selon la revendication 1, dans lequel la xylylène-diamine/les xylylène-diamines sont évaporées à 500 à 800 mbar.

4. Procédé selon l'une quelconque des revendications 1 à 3, dans lequel le temps de séjour moyen dans le circuit de l'évaporateur est de 10 à 60 minutes.

5. Procédé selon l'une quelconque des revendications 1 à 3, dans lequel le temps de séjour moyen dans le circuit de l'évaporateur est de 10 à 45 minutes.

6. Procédé selon l'une quelconque des revendications 1 à 5, dans lequel la température dans le circuit de pompage de l'évaporation est de 60 à 170 °C.

7. Procédé selon l'une quelconque des revendications 1 à 5, dans lequel la température dans le circuit de pompage de l'évaporation est de 60 à 150 °C.

8. Procédé selon l'une quelconque des revendications 1 à 7, dans lequel les vapeurs de XDA sont portées 20 à 50 K au-dessus de la température d'évaporation de la XDA à la pression donnée, puis mises en réaction avec du phosgène selon le procédé connu de phosgénation en phase gazeuse.

9. Procédé selon l'une quelconque des revendications 1 à 8, dans lequel un ou plusieurs stabilisateurs sont ajoutés à la XDA avant l'évaporation en concentrations de 50 à 2 000 ppm par rapport à la XDA.

10. Procédé selon la revendication 9, dans lequel les stabilisateurs sont des phénols à encombrement stérique et/ou des acides benzène-sulfoniques à substitution alkyle.

11. Procédé selon l'une quelconque des revendications 1 à 10, dans lequel une séparation de gouttelettes de XDA au moyen d'un séparateur de gouttelettes a lieu entre l'évaporation de la XDA et le surchauffage des vapeurs de XDA et/ou après le surchauffage des vapeurs de XDA.

12. Procédé selon la revendication 11, dans lequel le ou les appareils utilisés pour le surchauffage fonctionnent également en tant que séparateur de gouttelettes.

13. Diisocyanate de 1,3- et/ou 1,4-xylylène, fabriqué ou pouvant être fabriqué par un procédé selon l'une quelconque des revendications 1 à 12.

14. Utilisation de diisocyanate de 1,3- et/ou 1,4-xylylène selon la revendication 13 pour la fabrication de polyuréthanes ou polythiouréthanes transparents, notamment pour la fabrication de corps moulés en polyuréthanes ou polythiouréthanes transparents.

15. Corps moulé en polyuréthane ou polythiouréthane, fabriqué en utilisant du diisocyanate de 1,3- et/ou 1,4-xylylène selon la revendication 13.
